(19) Europäisches Patentamt
European Patent Office
Office européen des brevets

(11) Publication number: 0 364 707 A1

# (12) EUROPEAN PATENT APPLICATION

(21) Application number: 89116099.6

(51) Int. Cl.[5]: C12N 15/53

(22) Date of filing: 31.08.89

(30) Priority: 01.09.88 JP 216229/88

(43) Date of publication of application:
25.04.90 Bulletin 90/17

(84) Designated Contracting States:
CH DE FR GB LI NL

(71) Applicant: KIKKOMAN CORPORATION
339 Noda
Noda-shi(JP)

(72) Inventor: Tatsumi, Hiroki
65-1, Yanagisawa
Noda-shi(JP)
Inventor: Masuda, Tsutomu
190, West Street
Needham Massachusetts(US)
Inventor: Nakano, Eiichi
2-86, Kizora
Iwatsuki-shi(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

## (54) Method for producing luciferase.

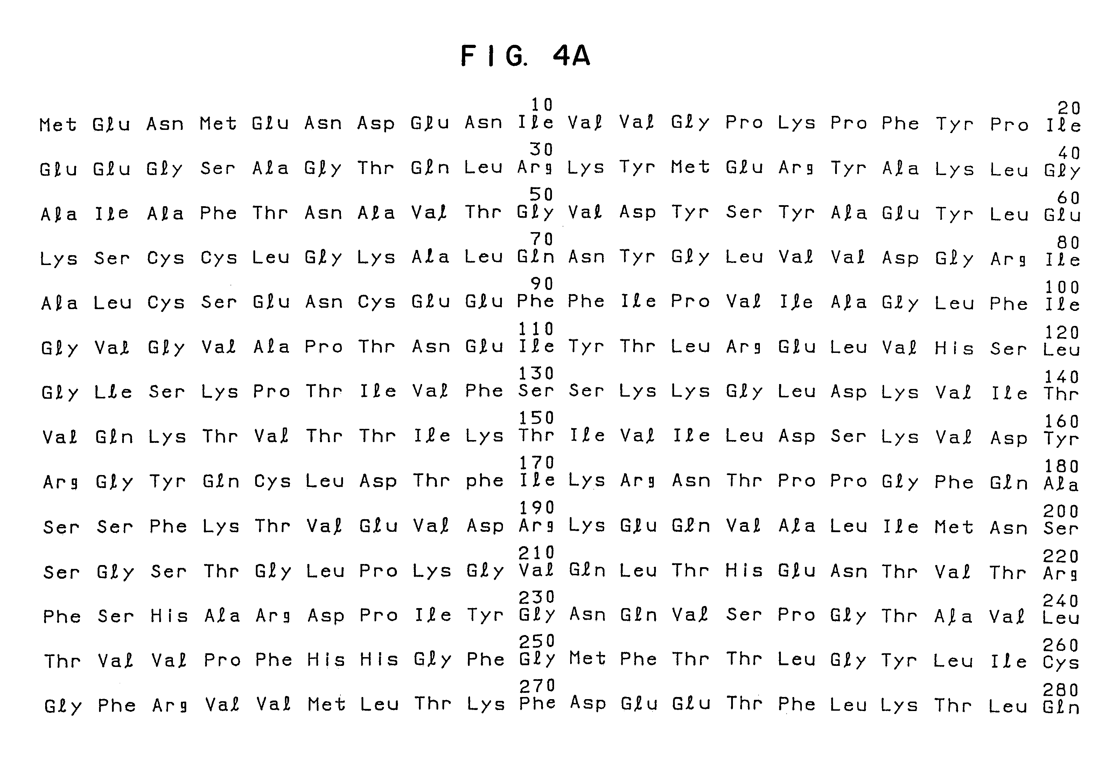

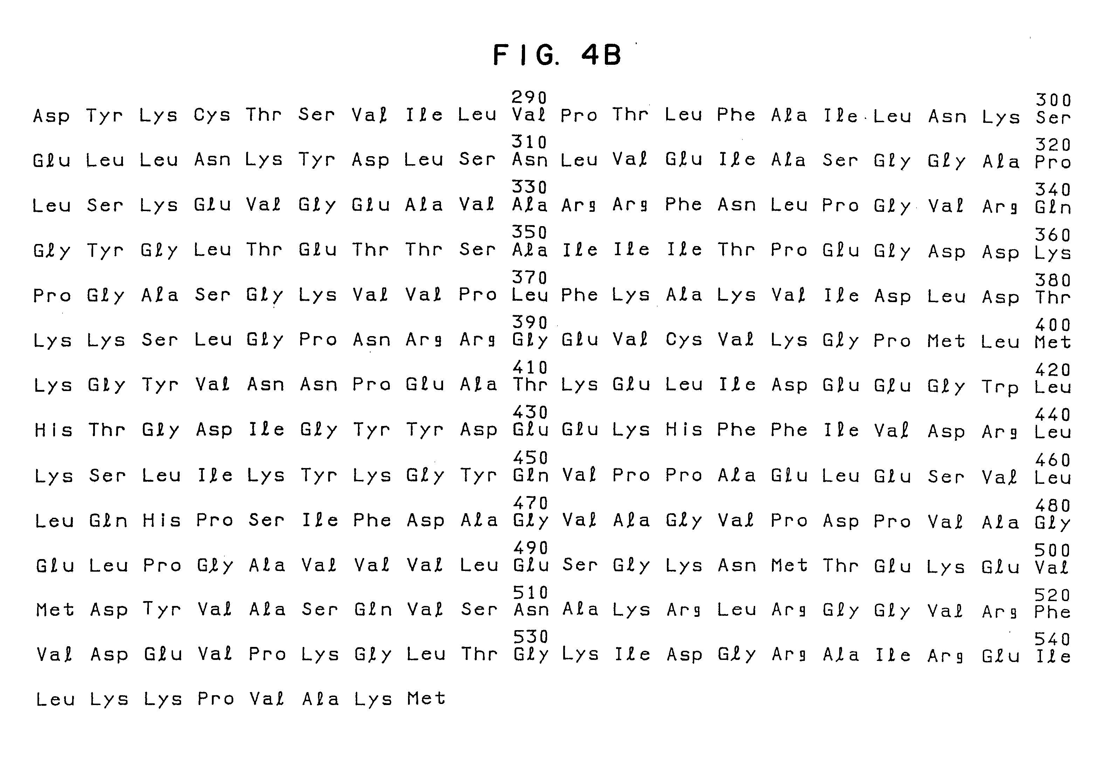

(57) A method for producing luciferase which comprises culturing in a medium a luciferase-producing microorganism belonging to the genus Escherichia and containing a recombinant DNA having incorporated into a vector DNA a luciferase gene encoding an amino acid sequence shown in Fig. 4, and collecting luciferase from the culture.

EP 0 364 707 A1

# METHOD FOR PRODUCING LUCIFERASE

## BACKGROUND OF THE INVENTION

### 1. Field of the Invention

The present invention relates to a method for producing luciferase derived from Luciola cruciata (GENJI firefly).

### 2. Description of the Prior Art

Luciferase from fireflies belonging to the genus Luciola is obtained simply by isolating and purifying from the collected fireflies belonging to the genus Luciola [Proc. Natl. Acad. Sci., 74 (7), 2799-2802 (1977)].

The luciferase is an enzyme which is an extremely useful enzyme, e.g., for ATP assay.

However, the luciferase described above is derived from insects and hence, for producing luciferase, fireflies belonging to the genus Luciola must be collected from the natural world or such fireflies must be cultivated and luciferase should be isolated and refined from the fireflies so that much time and labors are required for the production.

As a result of various investigations to solve the foregoing problems, the present inventors found that by obtaining a recombinant DNA having incorporated DNA containing a gene coding for luciferase into a vector DNA and culturing in a medium a luciferase-producing microorganism belonging to the genus Escherichia containing the recombinant DNA, luciferase could be efficiently produced in a short period of time, and have filed a patent application directed to this process (U.S. Patent Application Serial No. 224,445).

However, the gene encoding the luciferase described above is deleted of the nucleotide sequence corresponding to 9 amino acids from the N-terminus. Furthermore, the gene binds the N-terminal amino acid sequence, etc. of β-galactosidase derived from Escherichia coli at the deleted site. For these reasons, the total enzyme activity of the gene was not sufficiently satisfactory.

## SUMMARY OF THE INVENTION

Therefore, the present inventors have made extensive investigations to solve the foregoing problem and as a result, they have found that by culturing a microorganism containing a recombinant DNA having incorporated into a vector DNA a complete luciferase gene encoding an amino acid sequence shown below and capable of producing luciferase in a medium, there can be produced luciferase having an enzyme activity of about more than 10 times that in the process to which the above patent application was filed. The present invention has thus been accomplished.

10
Met Glu Asn Met Glu Asn Asp Glu Asn Ile
20
Val Val Gly Pro Lys Pro Phe Tyr Pro Ile
30
Glu Glu Gly Ser Ala Gly Thr Gln Leu Arg
40
Lys Tyr Met Glu Arg Tyr Ala Lys Leu Gly
50
Ala Ile Ala Phe Thr Asn Ala Val Thr Gly
60
Val Asp Tyr Ser Tyr Ala Glu Tyr Leu Glu
70
Lys Ser Cys Cys Leu Gly Lys Ala Leu Gln
80
Asn Tyr Gly Leu Val Val Asp Gly Arg Ile
90
Ala Leu Cys Ser Glu Asn Cys Glu Glu Phe
100
Phe Ile Pro Val Ile Ala Gly Leu Phe Ile
110
Gly Val Gly Val Ala Pro Thr Asn Glu Ile
120
Tyr Thr Leu Arg Glu Leu Val His Ser Leu
130
Gly Ile Ser Lys Pro Thr Ile Val Phe Ser
140
Ser Lys Lys Gly Leu Asp Lys Val Ile Thr
150
Val Gln Lys Thr Val Thr Thr Ile Lys Thr
160
Ile Val Ile Leu Asp Ser Lys Val Asp Tyr
170
Arg Gly Tyr Gln Cys Leu Asp Thr Phe Ile
180
Lys Arg Asn Thr Pro Pro Gly Phe Gln Ala
190
Ser Ser Phe Lys Thr Val Glu Val Asp Arg
200
Lys Glu Gln Val Ala Leu Ile Met Asn Ser
210
Ser Gly Ser Thr Gly Leu Pro Lys Gly Val
220
Gln Leu Thr His Glu Asn Thr Val Thr Arg

| | | | | | | | | | |
|---|---|---|---|---|---|---|---|---|---|
| | | | | | | | | | 230 |
| Phe | Ser | His | Ala | Arg | Asp | Pro | Ile | Tyr | Gly |
| | | | | | | | | | 240 |
| Asn | Gln | Val | Ser | Pro | Gly | Thr | Ala | Val | Leu |
| | | | | | | | | | 250 |
| Thr | Val | Val | Pro | Phe | His | His | Gly | Phe | Gly |
| | | | | | | | | | 260 |
| Met | Phe | Thr | Thr | Leu | Gly | Tyr | Leu | Ile | Cys |
| | | | | | | | | | 270 |
| Gly | Phe | Arg | Val | Val | Met | Leu | Thr | Lys | Phe |
| | | | | | | | | | 280 |
| Asp | Glu | Glu | Thr | Phe | Leu | Lys | Thr | Leu | Gln |
| | | | | | | | | | 290 |
| Asp | Tyr | Lys | Cys | Thr | Ser | Val | Ile | Leu | Val |
| | | | | | | | | | 300 |
| Pro | Thr | Leu | Phe | Ala | Ile | Leu | Asn | Lys | Ser |
| | | | | | | | | | 310 |
| Glu | Leu | Leu | Asn | Lys | Tyr | Asp | Leu | Ser | Asn |
| | | | | | | | | | 320 |
| Leu | Val | Glu | Ile | Ala | Ser | Gly | Gly | Ala | Pro |
| | | | | | | | | | 330 |
| Leu | Ser | Lys | Glu | Val | Gly | Glu | Ala | Val | Ala |
| | | | | | | | | | 340 |
| Arg | Arg | Phe | Asn | Leu | Pro | Gly | Val | Arg | Gln |
| | | | | | | | | | 350 |
| Gly | Tyr | Gly | Leu | Thr | Glu | Thr | Thr | Ser | Ala |
| | | | | | | | | | 360 |
| Ile | Ile | Ile | Thr | Pro | Glu | Gly | Asp | Asp | Lys |
| | | | | | | | | | 370 |
| Pro | Gly | Ala | Ser | Gly | Lys | Val | Val | Pro | Leu |
| | | | | | | | | | 380 |
| Phe | Lys | Ala | Lys | Val | Ile | Asp | Leu | Asp | Thr |
| | | | | | | | | | 390 |
| Lys | Lys | Ser | Leu | Gly | Pro | Asn | Arg | Arg | Gly |
| | | | | | | | | | 400 |
| Glu | Val | Cys | Val | Lys | Gly | Pro | Met | Leu | Met |
| | | | | | | | | | 410 |
| Lys | Gly | Tyr | Val | Asn | Asn | Pro | Glu | Ala | Thr |
| | | | | | | | | | 420 |
| Lys | Glu | Leu | Ile | Asp | Glu | Glu | Gly | Trp | Leu |

His Thr Gly Asp Ile Gly Tyr Tyr Asp Glu 430
Glu Lys His Phe Phe Ile Val Asp Arg Leu 440
Lys Ser Leu Ile Lys Tyr Lys Gly Tyr Gln 450
Val Pro Pro Ala Glu Leu Glu Ser Val Leu 460
Leu Gln His Pro Ser Ile Phe Asp Ala Gly 470
Val Ala Gly Val Pro Asp Pro Val Ala Gly 480
Glu Leu Pro Gly Ala Val Val Val Leu Glu 490
Ser Gly Lys Asn Met Thr Glu Lys Glu Val 500
Met Asp Tyr Val Ala Ser Gln Val Ser Asn 510
Ala Lys Arg Leu Arg Gly Gly Val Arg Phe 520
Val Asp Glu Val Pro Lys Gly Leu Thr Gly 530
Lys Ile Asp Gly Arg Ala Ile Arg Glu Ile 540
Leu Lys Lys Pro Val Ala Lys Met

That is, the present invention is directed to a method for producing luciferase which comprises culturing in a medium a luciferase-producing microorganism belonging to the genus Escherichia and containing a recombinant DNA having incorporated into a vector DNA a luciferase gene encoding an amino acid sequence shown below, and collecting luciferase from the culture.

Met Glu Asn Met Glu Asn Asp Glu Asn Ile 10

Val Val Gly Pro Lys Pro Phe Tyr Pro Ile 20

Glu Glu Gly Ser Ala Gly Thr Gln Leu Arg 30

Lys Tyr Met Glu Arg Tyr Ala Lys Leu Gly 40

Ala Ile Ala Phe Thr Asn Ala Val Thr Gly 50

Val Asp Tyr Ser Tyr Ala Glu Tyr Leu Glu 60

Lys Ser Cys Cys Leu Gly Lys Ala Leu Gln 70

Asn Tyr Gly Leu Val Val Asp Gly Arg Ile 80

Ala Leu Cys Ser Glu Asn Cys Glu Glu Phe 90

Phe Ile Pro Val Ile Ala Gly Leu Phe Ile 100

Gly Val Gly Val Ala Pro Thr Asn Glu Ile 110

Tyr Thr Leu Arg Glu Leu Val His Ser Leu 120

Gly Ile Ser Lys Pro Thr Ile Val Phe Ser 130

Ser Lys Lys Gly Leu Asp Lys Val Ile Thr 140

Val Gln Lys Thr Val Thr Thr Ile Lys Thr 150

Ile Val Ile Leu Asp Ser Lys Val Asp Tyr 160

Arg Gly Tyr Gln Cys Leu Asp Thr Phe Ile 170

Lys Arg Asn Thr Pro Pro Gly Phe Gln Ala 180

Ser Ser Phe Lys Thr Val Glu Val Asp Arg 190

Lys Glu Gln Val Ala Leu Ile Met Asn Ser 200

| | | | | | | | | | |
|---|---|---|---|---|---|---|---|---|---|
| | | | | | | | | | 210 |
| Ser | Gly | Ser | Thr | Gly | Leu | Pro | Lys | Gly | Val |
| | | | | | | | | | 220 |
| Gln | Leu | Thr | His | Glu | Asn | Thr | Val | Thr | Arg |
| | | | | | | | | | 230 |
| Phe | Ser | His | Ala | Arg | Asp | Pro | Ile | Tyr | Gly |
| | | | | | | | | | 240 |
| Asn | Gln | Val | Ser | Pro | Gly | Thr | Ala | Val | Leu |
| | | | | | | | | | 250 |
| Thr | Val | Val | Pro | Phe | His | His | Gly | Phe | Gly |
| | | | | | | | | | 260 |
| Met | Phe | Thr | Thr | Leu | Gly | Tyr | Leu | Ile | Cys |
| | | | | | | | | | 270 |
| Gly | Phe | Arg | Val | Val | Met | Leu | Thr | Lys | Phe |
| | | | | | | | | | 280 |
| Asp | Glu | Glu | Thr | Phe | Leu | Lys | Thr | Leu | Gln |
| | | | | | | | | | 290 |
| Asp | Tyr | Lys | Cys | Thr | Ser | Val | Ile | Leu | Val |
| | | | | | | | | | 300 |
| Pro | Thr | Leu | Phe | Ala | Ile | Leu | Asn | Lys | Ser |
| | | | | | | | | | 310 |
| Glu | Leu | Leu | Asn | Lys | Tyr | Asp | Leu | Ser | Asn |
| | | | | | | | | | 320 |
| Leu | Val | Glu | Ile | Ala | Ser | Gly | Gly | Ala | Pro |
| | | | | | | | | | 330 |
| Leu | Ser | Lys | Glu | Val | Gly | Glu | Ala | Val | Ala |
| | | | | | | | | | 340 |
| Arg | Arg | Phe | Asn | Leu | Pro | Gly | Val | Arg | Gln |
| | | | | | | | | | 350 |
| Gly | Tyr | Gly | Leu | Thr | Glu | Thr | Thr | Ser | Ala |
| | | | | | | | | | 360 |
| Ile | Ile | Ile | Thr | Pro | Glu | Gly | Asp | Asp | Lys |
| | | | | | | | | | 370 |
| Pro | Gly | Ala | Ser | Gly | Lys | Val | Val | Pro | Leu |
| | | | | | | | | | 380 |
| Phe | Lys | Ala | Lys | Val | Ile | Asp | Leu | Asp | Thr |
| | | | | | | | | | 390 |
| Lys | Lys | Ser | Leu | Gly | Pro | Asn | Arg | Arg | Gly |
| | | | | | | | | | 400 |
| Glu | Val | Cys | Val | Lys | Gly | Pro | Met | Leu | Met |

| | | | | | | | | | |
|---|---|---|---|---|---|---|---|---|---|
| | | | | | | | | | 410 |
| Lys | Gly | Tyr | Val | Asn | Asn | Pro | Glu | Ala | Thr |
| | | | | | | | | | 420 |
| Lys | Glu | Leu | Ile | Asp | Glu | Glu | Gly | Trp | Leu |
| | | | | | | | | | 430 |
| His | Thr | Gly | Asp | Ile | Gly | Tyr | Tyr | Asp | Glu |
| | | | | | | | | | 440 |
| Glu | Lys | His | Phe | Phe | Ile | Val | Asp | Arg | Leu |
| | | | | | | | | | 450 |
| Lys | Ser | Leu | Ile | Lys | Tyr | Lys | Gly | Tyr | Gln |
| | | | | | | | | | 460 |
| Val | Pro | Pro | Ala | Glu | Leu | Glu | Ser | Val | Leu |
| | | | | | | | | | 470 |
| Leu | Gln | His | Pro | Ser | Ile | Phe | Asp | Ala | Gly |
| | | | | | | | | | 480 |
| Val | Ala | Gly | Val | Pro | Asp | Pro | Val | Ala | Gly |
| | | | | | | | | | 490 |
| Glu | Leu | Pro | Gly | Ala | Val | Val | Val | Leu | Glu |
| | | | | | | | | | 500 |
| Ser | Gly | Lys | Asn | Met | Thr | Glu | Lys | Glu | Val |
| | | | | | | | | | 510 |
| Met | Asp | Tyr | Val | Ala | Ser | Gln | Val | Ser | Asn |
| | | | | | | | | | 520 |
| Ala | Lys | Arg | Leu | Arg | Gly | Gly | Val | Arg | Phe |
| | | | | | | | | | 530 |
| Val | Asp | Glu | Val | Pro | Lys | Gly | Leu | Thr | Gly |
| | | | | | | | | | 540 |
| Lys | Ile | Asp | Gly | Arg | Ala | Ile | Arg | Glu | Ile |
| Leu | Lys | Lys | Pro | Val | Ala | Lys | Met | | |

## BRIEF DESCRIPTION OF THE DRAWINGS

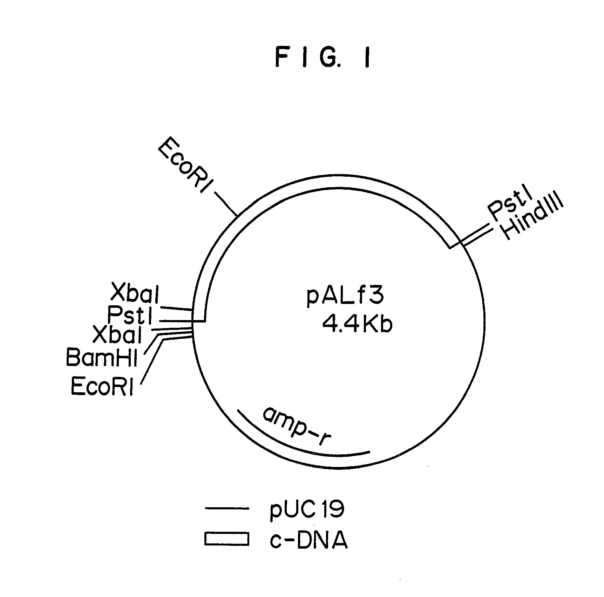

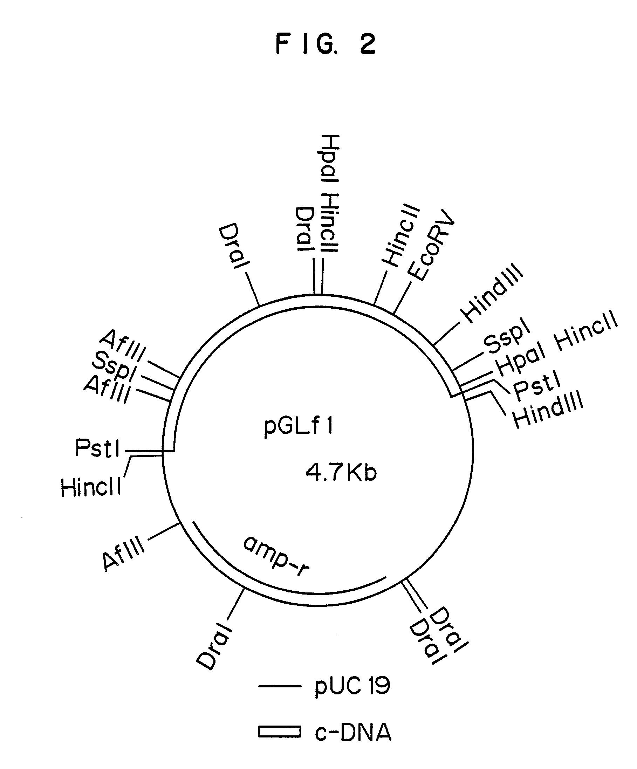

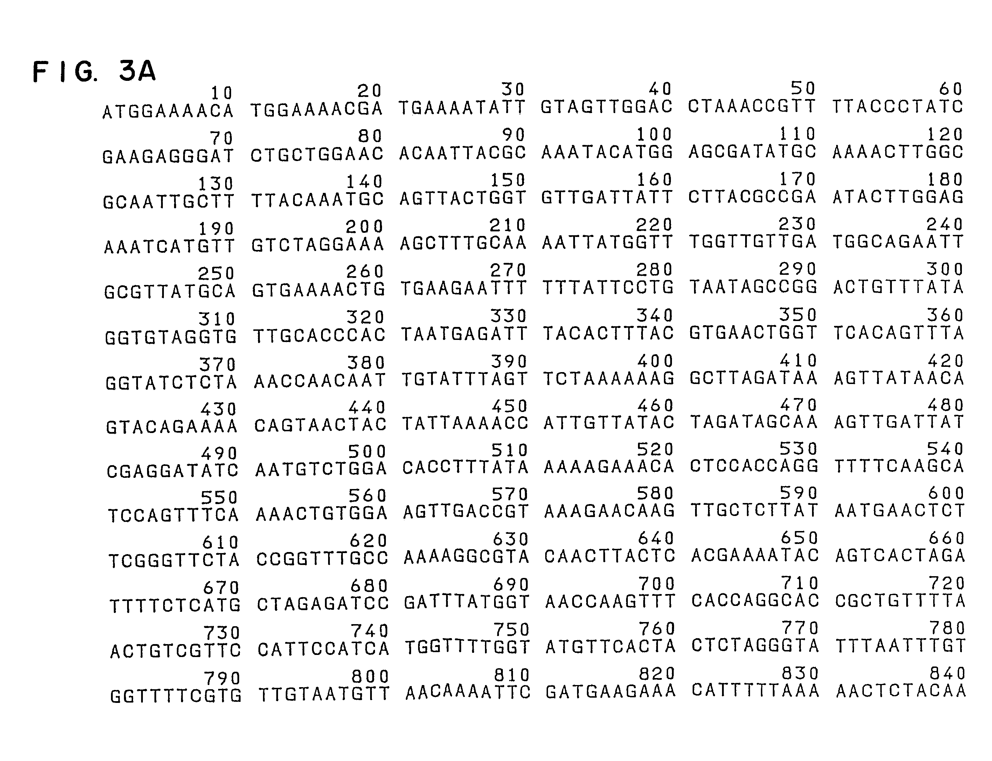

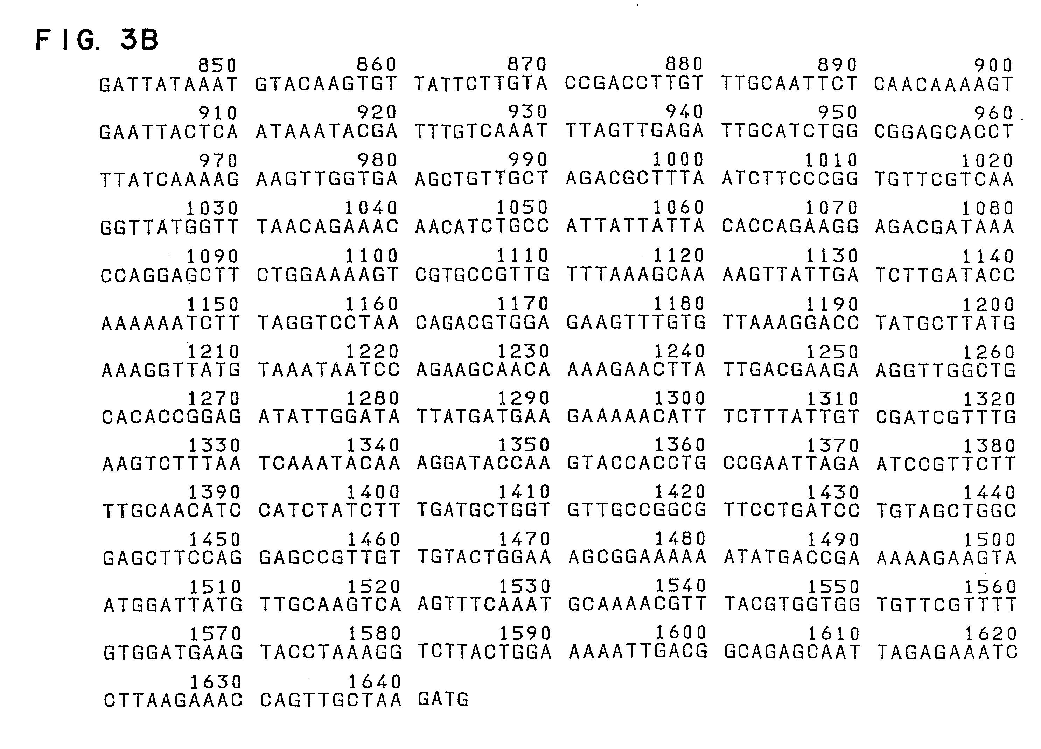

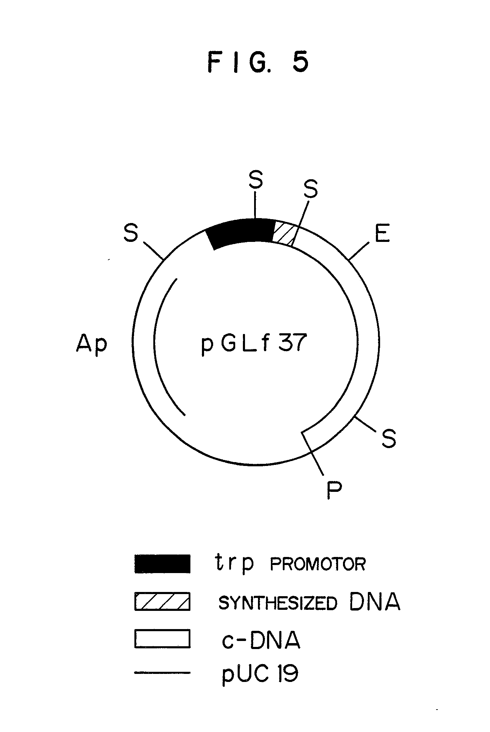

Fig. 1 shows a cleavage map of recombinant plasmid pALf3 DNA with restriction enzymes. Fig. 2 shows a cleavage map of recombinant plasmid pGLf1 DNA with restriction enzymes. Fig. 3 shows a nucleotide sequence of the luciferase gene used in the present invention. Fig. 4 shows an amino acid sequence of polypeptide translated from the luciferase gene used in the present invention. Fig. 5 shows a cleavage map of recombinant plasmid pGLf37 DNA with restriction enzymes.

## DESCRIPTION OF THE PREFERRED EMBODIMENTS

Hereafter the present invention is described in great detail.

In survey of DNA containing a gene coding for luciferase of Luciola cruciata, DNA containing a gene coding for luciferase derived from Photinus pyralis which is one of fireflies is used as a probe. Therefore, preparation of the DNA is firstly described below.

Preparation of m-RNA from the posterior portion of Photinus pyralis which is one of fireflies can be effected by methods described in, for example, Molecular Cloning, page 196, Cold Spring Harbor Laboratory (1982), Haruo Ozeki and Reiro Shimura, BUNSHI IDENGAKU JIKKENHO (Experimental Molecular Genetics), pages 66-67 (1983), etc.

Concentration of m-RNA coding for luciferase from the obtained m-RNA can be performed by a method described in, for example, Biomedical Research, 3, 534-540 (1982) or the like.

In this case, anti-luciferase serum to luciferase is used. This serum can be obtained by, for example, Yuichi Yamamura, MEN-EKI KAGAKU (Immunochemistry), pages 43-50 (1973), etc.

Synthesis of c-DNA from m-RNA coding for luciferase can be performed by methods described in, for example, Mol. Cell. Biol., 2, 161 (1982) and Gene, 25, 263 (1983).

Then, the thus obtained c-DNA is incorporated into, for example, plasmid pMCE10 DNA [plasmid prepared using plasmid pKN 305 [plasmid having a promoter of Escherichia coli tryptophane operator described in Agr. Biol. Chem., 50, 271 (1986)] and plasmid pMC 1843 [plasmid containing Escherichia coli β-galactosidase structural gene described in Methods in Enzymology, 100, 293-308 (1983)]], etc. to obtain various recombinant plasmid DNAs. Using these DNAs, transformation of Escherichia coli (E. coli) DH1 (ATCC 33849), Escherichia coli (E. coli) HB101 (ATCC 33694), etc. is effected by the method of Hanahan [DNA Cloning, 1, 109-135 (1985)] to obtain various transformants.

The recombinant plasmid DNAs possessed by the thus obtained transformants are plasmids wherein c-DNA has been incorporated in the middle of Escherichia coli β-galactosidase structural gene. A peptide encoded by c-DNA is expressed as a protein fused with β-galactosidase.

In order to detect c-DNA coding for luciferase from the various transformants described above, the transformants are cultured thereby to express cell protein. By determining if any protein crossing over anti-luciferase serum is present, the detection can be made. Methods descried in, for example, Agric. Biol. Chem., 50, 271 (1986) and Anal. Biochem., 112, 195 (1981), etc. can be used for the detection.

Next, after labeling c-DNA of incomplete luciferase with $^{32}P$ by the nick translation method [Molecular Cloning, pages 109-112, Cold Spring Harbor Laboratory (1982) and J. Mol. Biol., 113, 237-251 (1977)], using the colony hybridization method [Protein, Nucleic Acid & Enzyme, 26, 575-579 (1981)], an Escherichia coli strain having plasmid DNA containing Photinus pyralis luciferase c-DNA of 1.8 Kb can be obtained from a Photinus pyralis-derived c-DNA library prepared using plasmid pUC19 DNA (manufactured by Takara Shuzo Co., Ltd.) as a vector.

To obtain DNA containing the gene coding for luciferase derived from Photinus pyralis from the thus obtained recombinant plasmid DNA, restriction enzymes, e.g., Eco RI and Cla I, are acted on the plasmid DNA at temperatures of 30 to 40° C, preferably at 37° C, for 1 to 24 hours, preferably 2 hours; the solution obtained after completion of the reaction is subjected to agarose gel electrophoresis [which is described in Molecular Cloning, page 150, Cold Spring Harbor Laboratory (1982)] to obtain DNA containing the gene coding for luciferase derived from Photinus pyralis.

Next, preparation of the luciferase gene and the like in accordance with the present invention are described below.

Firstly, the source from which the gene coding for luciferase is derived may be any source and, mention may be made of, for example, Luciola cruciata (GENJI firefly), etc. Particularly preferred is the posterior portion of this firefly.

And preparation of m-RNA from the posterior portion of the firefly and synthesis of c-DNA from m-RNA can be conducted, for example, in quite the same manner as in the preparation of m-RNA of Photinus pyralis and synthesis of c-DNA described above.

Then, the thus obtained c-DNA is incorporated into a vector DNA, for example, plasmid pUC19 DNA (manufactured by Takara Shuzo Co., Ltd.), etc. to obtain various recombinant plasmid DNAs. Using these DNAs, transformation of Escherichia coli (E. coli) DH1 (ATCC 33849), Escherichia coli (E. coli) HB101 (ATCC 33694), etc. is effected by the method of Hanahan [DNA Cloning, 1, 109-135 (1985)] to obtain various transformants.

Next, after labeling DNA containing the gene coding for luciferase derived from Photinus pyralis with $^{32}P$ by the nick translation method [Molecular Cloning, pages 109-112, Cold Spring Harbor Laboratory (1982) and J. Mol. Biol., 113, 237-251 (1977)], using the colony hybridization method [protein, Nucleic Acid & Enzyme, 26, 575-579 (1981)], an Escherichia coli strain having plasmid DNA containing Luciola cruciata luciferase c-DNA of 2.0 Kb can be obtained from a Luciola cruciata-derived c-DNA library prepared using plasmid pUC19 DNA (manufactured by Takara Shuzo Co., Ltd.) as a vector.

Then, the Luciola cruciata-derived c-DNA of 2.0 Kb encoding luciferase is treated with restriction enzyme Ssp I to give c-DNA fragment of 1.7 Kb, namely, luciferase gene deleted of the nucleotide sequence encoding 9 amino acids from the N-terminus. The recombinant DNA bearing the nucleotide sequence completely encoding Luciola cruciata-derived luciferase gene can be obtained from the c-DNA fragment, promoter [e.g., trp promoter derived from E. coli, etc.], vector DNA and the nucleotide sequence encoding 9 amino acids from the N-terminus of luciferase gene, using restriction enzymes, for example, Ssp I (manufactured by New England Biolab Co.), or the like and T4 DNA ligase (manufactured by Takara Shuzo Co., Ltd.), etc.

As the vector DNA described above, any vector DNA may be used and thre are mentioned plasmid vector DNA, bacteriophage vector DNA, etc. Specific examples are pUC18 (manufactured by Takara Shuzo Co., Ltd.), pUC19 (manufactured by Takara Shuzo Co., Ltd.), λc I857, h80att $\lambda sRI\lambda_3^0$ $sRI\lambda_2^0$ $sRI\lambda_1^0$ (described

in Japanese Patent Publication KOKOKU No. 61-37917), etc.

Using the thus obtained novel recombinant DNA, microorganism belonging to the genus Escherichia, for example, Escherichia coli JM101 (ATCC 33876) or the like, is transformed by the method of Cohen et al. [J. Bac., 119, 1072-1074 (1974)] or transfected by the method described in Molecular Cloning, pages 256-268, Cold Spring Harbor Laboratory (1982)] to give luciferase-producing microorganism belonging to the genus Escherichia containing the novel recombinant DNA having inserted the luciferase-encoding gene into vector DNA.

To obtain a purified novel recombinant DNA from the thus obtained micrcorganism, there is used, for example, a method described in Proc. Natl. Acad. Sci., 62, 1159-1166 (1969), etc.

Next, the microorganism described above is cultured in a medium and luciferase is collected from the culture.

Any medium may be used so long as it is used for culture of microorganism belonging to, for example, the genus Escherichia. Examples include 1% (W/V) of trypton, 0.5% (W/V) of yeast extract, 0.5% (W/V) of NaCl, etc.

Temperature for the cultivation is between 30 and 40° C, preferably about 37° C and a time period for the cultivation is, for example, 4 to 8 hours, preferably about 4 hours.

The cells are collected from the culture by centrifugation at 8,000 r.p.m. for about 10 minutes. The obtained cells are homogenized by the method described in, for example, Methods in Enzymology, 133, 3-14 (1986) to obtain a crude enzyme solution.

The crude enzyme solution may be usable as it is; if necessary and desired, the crude enzyme solution can be purified by fractionation with ammonium sulfate, hydrophobic chromatography, for example, using Butyl Toyo Pearl 650 C, etc., gel filtration using, e.g., Ultrogel AcA 34, etc. thereby to give purified luciferase.

Physicochemical properties of the thus obtained luciferase are as described below..

(1) Action:

The enzyme catalyzes the oxidation of luciferin by an oxygen molecule, as shown by the enzymatic reaction equation:

$$\text{Luciferin} + \text{ATP} + O_2 \xrightarrow{\text{Present enzyme}}$$

$$\text{Oxyluciferin} + \text{AMP} + \text{Pyrophosphoric acid} + CO_2 + \text{light}$$

(2) Substrate specificity:

The enzyme does not act on ADP, CTP, UTP and GTP.

(3) Optimum pH, and pH range for stability:

The optimum pH is 8.0 to 9.5 as measured by carrying out the reaction by the use of luciferin as a substrate at various pH values of 25 mM glycylglycine buffer solution in the range of 6.5 to 11.5 and at a temperature of 30° C, and measuring the quantity of light (the number of photons) emitted in 20 seconds. The stable pH range of the enzyme is 6.5 to 9.0 as measured by adding the enzyme to each of buffer solutions [pH 4.6 - 8.0: 25 mM phosphate buffer; pH 8.0 - 11.5: 25 mM glycine.sodium chloride-sodium hydroxide buffer solutions), each of which contains ammonium sulfate to 10% saturation] containing luciferin, and allowing the enzyme to act at a temperature of 0° C for 4 hours.

(4) Measurement of titer:

A luciferin mixed solution is prepared by mixing 8 ml of 25 mM glycylglycine buffer solution (pH 7.8),

0.5 ml of a magnesium sulfate solution [a solution prepared by adding magnesium sulfate to 25 mM glycylglycine buffer solution (pH 7.8) in a mangesium sulfate concentration of 0.1 M] and 0.8 ml of a luciferin solution [a solution prepared adding luciferin to 25 mM glycylglycine buffer solution (pH 7.8) in a luciferin concentration of 1 mM].

Into a mixture of 400 µl of the luciferin mixed solution thus obtained and 10 µl of luciferase to be assayed is poured 80 µl of an ATP solution [a solution prepared by adding ATP to 25 mM glycylglycine buffer solution (pH 7.8) in an ATP concentration of 10 mM]. Simultaneously with the pouring, the number of photons generated is measured by adding up for 20 seconds by means of a luminometer (LUMINESCENCE READER BLR-201, manufactured by ALKOA Co., Ltd.).

(5) Ragne of temperature suitable for activity:

When the reaction is carried out at pH 7.8 at each temperature and the quantity of light (the number of photons) emitted in 20 seconds is measured, the suitable temperature for the action ranges from 0° to 50° C.

(6) Conditions for inactivation by pH, temperature, etc.

(a) Conditions for inactivation by pH

At pH values of 5.0 or lower and 11.0 or higher, the enzyme is completely inactivated 4 hours after.

(b) Conditions for inactivation by temperature

At pH of 7.8, the enzyme is completely inactivated by heat treatment at a temperature of 50° C for 15 minutes.

As is clear from the foregoing description, according to the present invention, luciferase can be efficiently produced in an extremely short period of time by culturing the microorganism belonging to the genus Escherichia which contains the recombinant DNA having incorporated therein the gene completely encoding luciferase derived from Luciola cruciata. Therefore, the present invention is extremely useful from an industrial point of view.

Hereafter the present invention will be described in more detail by referring to the examples below.

Examples

In Items 1 to 10 below, preparation of DNA containing a gene coding for luciferase of Photinus pyralis as one of fireflies (this DNA is used as a probe upon survey of DNA containing a gene coding for luciferase of Luciola cruciata) is described.

1. Preparation of m-RNA

Using a mortar and a pestle, 1 g of the dry posterior portion (manufactured by Sigma Co., Ltd.) of Photinus pyralis as one of fireflies was thoroughly ground, to which 5 ml of dissolution buffer [20 mM Tris-hydrochloride buffer (pH 7.4)/10 mM NaCl/3 mM magnesium acetate/5% (W/V) sucrose/1.2 % (V/V) tryton X-100/10 mM vanadyl nucleoside complex (manufactured by New England Biolab Co., Ltd.)] was added. The mixture was further ground as in the manner described above to give a solution containing the ground posterior portion of Photinus pyralis.

In a cup blender (manufactured by Nippon Seiki Seisakusho) was charged 5 ml of the thus obtained solution. After treating at 5,000 r.p.m. for 5 minutes, 12 ml of guanidine isothiocyanate solution (6M guanidine isothiocyanate/37.5 mM sodium citrate (pH 7.0)/0.75% (W/V) sodium N-lauroylsarcosine/0.15 M β-mercaptoethanol) was added to the system. The mixture was treated with the blender described above at 3,000 r.p.m. for 10 minutes. The resulting solution was filtered using a threefold gauze to give the filtrate.

The filtrate was gently poured in layers onto 4 tubes for ultracentrifuging machine (manufactured by Hitachi Koki Co., Ltd.) in which 1.2 ml each of 5.7 M cesium chloride solution had previously be laid in layers. Using the ultracentrifuging machine (manufactured by Hitachi Koki Co., Ltd., SCP55H), centrifugation was performed at 30,000 r.p.m. for 16 hours to give precipitates.

The obtained precipitates were washed with cold 70% (V/V) ethanol and suspended in 4 ml of 10 mM Tris buffer [10 mM Tris-hydrochloride (pH 7.4)/5 mM EDTA/1% sodium dodecylsulfate]. The equal amount of a mixture of n-butanol and chloroform in 4 : 1 (volume ratio) was added to the mixture to perform extraction. The extract was centrifuged at 3,000 r.p.m. for 10 minutes in a conventional manner to separate into the aqueous phase and the organic solvent phase. To the organic solvent phase was added 4 ml of 10 mM Tris buffer described above. The procedure for the extraction and separation described above was repeated twice. To the aqueous phase obtained were added a 1/10 amount of 3 M sodium acetate (pH 5.2) and a 2-fold amount of cold ethanol were added. After allowing to stand at a temperature of -20° C for 2 hours, the mixture was centrifuged at 8,000 r.p.m. for 20 minutes in a conventional manner to precipitate RNA. The obtained RNA was dissolved in 4 ml of water. After the operation for precipitation with ethanol described above was carried out, the obtained RNA was dissolved in 1 ml of water to give 3.75 mg of RNA.

By repeating the foregoing procedure again, 7 mg in total of RNA was produced. To select m-RNA from the RNA, 7 mg of RNA was subjected to oligo(dT)-cellulose (manufactured by New England Biolab Co., Ltd.) column chromatography.

As the column, 2.5 ml of Terumo syringe (manufactured by Terumo Co., Ltd.) was used. After 0.5 g of resin was swollen with elution buffer [10 mM Tris-hydrochloride buffer (pH 7.6)/1 mM DETA/0.1% (W/V) sodium dodecylsulfate], the resin was packed in the column and equilibrated with binding buffer [10 mM Tris-hydrochloride (pH 7.6)/1 mM DETA/0.4 M NaCl/0.1% (W/V) sodium dodecylsulfate].

To 7 mg of RNA was added the same amount of buffer [10 mM Tris-hydrochloride (pH 7.6)/1 mM DETA/0.8 M NaCl/0.1% (W/V) sodium dodecylsulfate]. The mixture was heat-treated at a temperature of 65° C for 10 minutes and then quenched in ice water. After subjecting to oligo(dT)-celluose column, the resin was washed with binding buffer to completely wash unbound r-RNA and t-RNA out. Further m-RNA was eluted with eluting buffer to give 40 µg of luciferase m-RNA.

2. Concentration of luciferase m-RNA

Next, the luciferase m-RNA was concentrated by sucrose density gradient centrifugation.

Sucrose density gradient of 10 to 25% (W/V) was produced by charging 0.5 ml of 40% (W/V) sucrose solution [50 mM Tris-hydrochloride buffer (pH 7.5)/20 mM NaCl/1 mM EDTA/40% (W/V) sucrose] in a polyaroma tube for Rotar SW41 manufactured by Beckmann Co., Ltd., laying 2.4 ml each of 25% (W/V), 20% (W/V), 15% (W/V) and 10% (W/V) of the sucrose solution in layers and allowing to stand the system at a temperature of 4° C for 24 hours. To the sucrose density gradient, 30 µg of m-RNA was laid to form a layer. Using SW41 Rotar manufactured by Beckmann Co., Ltd., centrifugation was conducted at 30,000 r.p.m. at a temperature of 18° C for 18 hours in a conventional manner. After the centrifuging operation, fractionation was performed by 0.5 ml each and m-RNA was recovered by the ethanol precipitation method. The m-RNA was dissolved in 10 µl of water.

Next, protein encoded by the m-RNA was examined, whereby the fraction concentrated on m-RNA of luciferase was identified. One microliter of the fractionated RNA, 9 µl of rabbit reticular erythrocyte lysate (manufactured by Amersham Co., Ltd.) and 1 µl of [$^{35}$S] methionine (manufactured by Amersham Co., Ltd.) were mixed and reacted at a temperature of 30° C for 30 minutes. To the reaction mixture was added 150 µl of NET buffer [150 mM NaCl/5 mM EDTA/0.02% (W/V) $NaN_3$/20 mM Tris-hydrochloride buffer (pH 7.4)-/0.05% (W/V) Nonidet P-40 (manufactured by Besesda Research Laboratories Co., Ltd., surface active agent)] and, 1 µl of anti-luciferase serum (produced as will be later described) was added to the mixture. After allowing to stand at a temperature of 20° C for 30 hours, 10 mg of Protein A Sepharose (manufactured by Pharmacia Fine Chemicals Inc.) was added to the mixture. The resulting mixture was then centrifuged at 12,000 r.p.m. for a minute in a conventional manner to recover the resin.

The recovered resin was washed three times with 200 µl of NET buffer. To the resin was added 40 µl of sample buffer for SDS-PAGE [62.5 mM Tris-hydrochloride buffer (pH 6.8)/10% (V/V) glycerol/2% (W/V) sodium dodecylsulfate/5% (V/V) mercaptoethanol/0.02% (W/V) bromophenol blue]. The mixture was boiled at a temperature of 100° C for 3 minutes and centrifuged at 12,000 r.p.m. for a minute in a conventional manner to recover the supernatant. The whole amount was applied onto 7.5% (W/V) sodium dodecylsulfate-polyocrylamide gel.

Gel electrophoresis was performed by the method of Laemmli [Nature, 227, 680 (1970)]. After the

electrophoresis, the gel was immersed in 10% (V/V) acetic acid for 30 minutes to immobilize protein. Then, the gel was immersed in water for 30 minutes and further immersed in 1 M sodium salicylate solution for 30 minutes and then dried to give a dry gel. The dry gel was subjected to fluorography using an X ray film (manufactured by Fuji Photo Film Co., Ltd.; RX).

By the foregoing procedure, the band of luciferase protein was recognized on the X ray film only in the case of using the RNA from the fraction in which the luciferase m-RNA was present and, the fraction wherein the luciferase m-RNA was concentrated could be identified.

3. Production of anti-serum

Rabbit anti-luciferase serum to purified luciferase was produced by the following method.

A luciferase solution having a 3.2 mg/ml concentration [solution obtained by dissolving luciferase manufactured by Sigma Co., Ltd. in 0.5 M glycylglycine solution (pH 7.8)], 0.7 ml, was suspended in an equal amount of Freund's complete adjuvant. 2.24 mg of the suspension was administered as an antigen to the palm of Japanese white rabbit weighing 2 kg as an antigen. After feeding for 2 weeks, the same amount of antigen as in the initial amount was intracutaneously administered to the back. After feeding for further one week, similar procedure was performed. Further one week after feeding, whole blood was collected.

The obtained blood was allowed to stand at a temperature of 4° C for 18 hours and then centrifuged at 3,000 r.p.m. for 15 minutes in a conventional manner to give anti-luciferase serum as the supernatant.

4. Synthesis of c-DNA

Synthesis of c-DNA was carried out using a kit manufactured by Amersham Co., Ltd.

Using 2 µg of m-RNA obtained as described above, synthesis of c-DNA was carried out in accordance with the methods described in Mol. Cell Biol., 2, 161 (1982) and Gene, 25, 263 (1983): As the result, 300 ng of double stranded c-DNA was obtained.

This c-DNA, 150 ng, was dissolved in 7 µl of TE buffer [10 mM Tris-hydrochloride buffer (pH 7.5)/1 mM EDTA]. To the solution were added, respectively, 11 µl of a mixture [280 mM sodium cacodylate (pH 6.8)-/60 mM Tris-hydrochloride buffer (pH 6.8)/2 mM cobalt chloride] and 3.8 µl of a tailing mixture [7.5 µl of 10 mM dithiothreitol/1 µl of 10 ng/ml poly(A)/2 µl of 5mM dCTP/110 µl of water]. Furthermore, 29 units of terminal transferase (manufactured by Boehringer Mannheim GmbH) was added to the mixture. After reacting at a temperature of 30° C for 10 minutes, 2.4 µl of 0.25 M EDTA and 2.4 µl of 10% (W/V) sodium dodecylsulfate were added to the mixture to terminate the reaction.

The solution in which the reaction had been discontinued was subjected to a treatment for removing protein using 25 µl of water-saturated phenol. Then, 25 µl of 4 M ammonium acetate and 100 µl of cold ethanol were added to the recovered aqueous phase, respectively. The mixture was allowed to stand at a temperature of -70° C for 15 minutes and centrifuged at 12,000 r.p.m. for 10 minutes to recover c-DNA. The c-DNA was dissolved in 10 µl of TE buffer to give a c-DNA solution.

As described above, 100 ng of the c-DNA with the deoxycytidine tail was obtained.

5. Production of recombinant plasmid pMCE10 DNA used in vector

E. coli W3110 strain (ATCC 27325), plasmid pKN305 DNA produced by the method described in T. Masuda et al., Agricultural Biological Chemistry, 50, 271-279 (1986) using plasmid pBR325 (manufactured by BRL Co.) and plasmid pBR322 DNA (manufactured by Takara Shuzo Co., Ltd.) and pMC1403-3 DNA (described in Japanese Patent Application KOKAI No. 61-274683) were added by 1 µg each to 10 µl of a mixture [50 mM Tris-hydrochloride buffer (pH 7.5)/10 mM $MgCl_2$/100 mM NaCl/1 mM dithiothreitol]. Further, 2 units each of Hind III and Sal I (both manufactured by Takara Shuzo Co., Ltd.) were added to the mixture. By reacting at a temperature of 37° C for an hour, a cleavage treatment was effected. Extraction with phenol and precipitation with ethanol were conducted in a conventional manner to give precipitates. The precipitates were dissolved in 10 µl of ligation buffer [20 mM $MgCl_2$/66 mM Tris-hydrochloride buffer (pH 7.6)/1 mM ATP/15 mM dithiothreitol] to give a solution. Furthermore, 1 unit of T4 DNA ligase (manufactured by Takara Shuzo Co., Ltd.) was added thereto to perform ligation at a temperature of 20° C for 4 hours. Then, using this reaction solution, E. coli JM101 (ATCC 33876) was transformed according to the transformation method described in J. Bacteriology, 119, 1072-1074 (1974). By examination of chemical

resistance (ampicillin resistance and tetracycline sensitivity) and β-galactosidase activity, a transformant was obtained. Recombinant plasmid DNA contained in the strain was named pMCE10. E. coli JM101 strain containing this recombinant plasmid DNA pMCE10 DNA was cultured in medium composed of 1% (W/V) of trypton, 0.5% (W/V) of yeast extract and 0.5% (W/V) of NaCl at a temperature of 37° C for 16 to 24 hours. Twenty milliliters of the thus obtained culture solution of E. coli JM101 (pMCE10) was inoculated on 1 liter of the medium followed by shake culture at a temperature of 37° C for 3 hours. After the addition of 0.2 g of chloramphenicol, cultivation was conducted at the same temperature for further 20 hours to give a culture solution.

Next, the culture solution was centrifuged at 6,000 r.p.m. for 10 minutes in a conventional manner to give 2 g of wet cells. After the cells were suspended in 20 ml of 350 mM Tris-hydrochloride buffer (pH 8.0) containing 25% (W/V) sucrose, 10 mg of lysozyme, 8 ml of 0.25 M EDTA solution (pH 8.0) and 8 ml of 20% (W/V) sodium dodecylsulfate were added to the suspension, respectively. The mixture was kept at a temperature of 60° C for 30 minutes to give a lysate solution.

To the lysate solution was added 13 ml of 5 M NaCl solution. The mixture was treated at a temperature of 4° C for 16 hours and then centrifuged at 15,000 r.p.m. in a conventional manner to give an extract. The extract was subjected to the phenol extraction and the ethanol precipitation in a conventional manner to give precipitates.

Then, the precipitates were dried under reduced pressure in a conventional manner and dissolved in 10 mM Tris-hydrochloride buffer (pH 7.5) containing 1 mM EDTA. To the solution were further added 6 g of cesium chloride and 0.2 ml of ethydium bromide solution (10 mg/ml). The resulting mixture was subjected to an equilibrated density gradinent centrifugation treatment using a ultracentrifuging machine at 39,000 r.p.m. for 42 hours in a conventional manner thereby to isolate recombinant plasmid pMCE10 DNA. After ethydium bromide was removed using n-butanol, dialysis was performed to 10 mM Tris-hydrochloride buffer (pH 7.5) containing 1 mM EDTA to give 500 μg of purified recombinant plasmid pMCE10 DNA.

6. Production of vector DNA

The thus obtained recombinant plasmid pMCE10 DNA, 15 μg, was dissolved in 90 μl of TE buffer described in Item 4. After 10 μl of Med buffer [100 mM Tris-hydrochloride buffer (pH 7.5)/100 mM $MgCl_2$/10 mM dithiothreitol/500 mM NaCl] was added to the solution, 30 units of restriction enzyme Acc I (manufactured by Takara Shuzo Co., Ltd.) was further added to the mixture. A cleavage treatment was conducted at a temperature of 37° C for an hour to give the cleavage product. To the cleavage product was added 100 μl of water-saturated phenol, whereby protein was removed. Then, the aqueous phase was recovered and a 1/10-fold amount of 3 M sodium acetate (pH 7.5) and a 2-fold amount of cold ethanol were added to the aqueous phase. After allowing to stand at a temperature of -70° C for 15 minutes, the mixture was centrifuged at 12,000 r.p.m. for 10 minutes to recover DNA.

This DNA was dissolved in 10 μl of TE buffer and 15 μl of a mixture [280 mM sodium cacodylate (pH 6.8)/60 mM Tris-hydrochloride buffer (pH 6.8)/2 mM cobalt chloride] was added to the solution. Then, 5 μl of a tailing solution mixture (described in Item 4) (5 mM dGTP was used instead of 5 mM dCTP) was further added to the mixture. Furthermore, 5 units of terminal transferase (manufactured by Takara Shuzo Co., Ltd.) was added to react at a temperature of 37° C for 15 minutes. By post-treatment in a manner similar to the c-DNA tailing reaction described in Item 4. DNA with the deoxyguanosine tail at the Acc I site of recombinant plasmid pMCE10 DNA was produced.

On the other hand, DNA with the deoxyguanosine tail at the Pst I site of plasmid pUC19 DNA was also produced at the same time.

To a solution of 30 μg of plasmid pUC19 DNA (manufactured by Takara Shuzo Co., Ltd.) in 350 μl of TE buffer were added 40 μl of Med buffer and 120 units of restriction enzyme Pst I (manufactured by Takara Shuzo Co., Ltd.). After a cleavage treatment at a temperature of 37° C for an hour, DNA was recovered by the phenol treatment for removing protein and ethanol precipitation in a conventional manner.

The obtained DNA was dissolved in 35 μl of TE buffer. To the solution were added 50 μl of a mixture [280 mM sodium cacodylate (pH 6.8)/60 mM Tris-hydrochloride buffer (pH 6.8)/1 mM cobalt chloride], 19 μl of the tailing mixture (containing dGTP instead of dCTP) described in Item 4 and 60 units of terminal transferase (manufactured by Takara Shuzo Co., Ltd.). After reacting at a temperature of 37° C for 10 minutes, DNA was recovered by the phenol treatment and ethanol precipitation in a conventional manner.

7. Annealing and transformation

The thus synthesized c-DNA, 15 ng, and 200 ng of two vector DNAs obtained by the procedure described above were dissolved in 35 µl of annealing buffer [10 mM Tris-hydrochloride buffer (pH 7.5)/100 mM NaCl/1 mM EDTA]. The solution was allowed to stand at a temperature of 65 °C for 2 minutes, at a temperature of 46 °C for 2 hours, at a temperature of 37 °C for an hour and at a temperature of 20 °C for 18 hours thereby to anneal c-DNA and vector DNAs.

Using the annealed DNA, E. coli DH1 strain (ATCC 33849) was transformed by the method of Hanahan [DNA Cloning, 1, 109-135 (1985)] to produce a c-DNA bank containing plasmid pUC19 DNA and recombinant plasmid pMCE10 DNA as vectors, respectively.

8. Survey of luciferase c-DNA

The Acc I site of recombinant plasmid pMCE10 DNA is present at a site which codes for E. coli β-galactosidase gene. Therefore, c-DNA incorporated into this site forms a fused protein with β-galactosidase. Furthermore, a promoter of β-galactosidase gene of the recombinant plasmid pMCE10 DNA has been converted into a promoter of E. coli tryptophan gene, as described above.

96 colonies of c-DNA having recombinant plasmid pMCE10 DNA as a vector were shake cultured in 10 ml of M9 Casamino acid medium [Molecular Cloning, 440-441, Cold Spring Harbor Laboratory (1982)] supplemented with thiamine (10 µg/ml) at a temperature of 37 °C for 10 hours. After collecting the cells in a conventional manner, the cells were suspended in 200 µl of sample buffer for SDS-PAGE described in Item 2. The suspension was boiled at a temperature of 100 °C for 5 minutes.

This suspension, 40 µl, was subjected to electrophoresis in a conventional manner using 7.5% (W/V) polyacrylamide gel. After completion of the electrophoresis, the protein developed on the gel was transferred onto a nitrocellulose filter by the western blot method [Anal. Biochem., 112, 195 (1981)]. This nitrocellulose filter was stained with anti-luciferase serum using immune blot assay kit (manufactured by Biorad Co.). The method was performed in accordance with the instruction of Biorad Co.

That is, the nitrocellulose filter was shaken in 100 ml of blocking solution [a solution obtained by dissolving 3% (W/V) gelatin in TBS buffer [20 mM Tris-hydrochloride buffer /500 mM NaCl (pH 7.5)] at a temperature of 25 °C for 30 minutes. Next, this nitrocellulose filter was transferred into 25 ml of primary antibody solution [solution obtained by dissolving 1% (W/V) gelatin in TBS buffer and diluting luciferase anti-serum with the resulting solution] and shaken at a temperature of 25 °C for 90 minutes, which was then transferred into 100 ml Tween-20 washing solution [solution obtained by dissolving 0.05% (W/V) Tween-20 in TBS buffer] and shaken at a temperature of 25 °C for 10 minutes. This procedure was repeated twice. Then, the thus obtained nitrocellulose filter was transferred into 60 ml of secondary antibody solution [solution obtained by dissolving anti-rabbit antibody labeled with horse raddish peroxidase (manufactured by Biorad Co.) with a solution of 1% (W/V) gelatin in TBS buffer to 3000-fold (V/V)]. After shaking at a temperature of 25 °C for 60 minutes, the nitrocellulose filter was washed with 100 ml of Tween-20 washing solution. The procedure described above was repeated twice. The thus obtained nitrocellulose filter was transferred into 120 ml of color forming solution [solution obtained by mixing a solution of 60 mg of 4-chloro-1-naphthol in 20 ml of cold methanol and a solution of 60 µl of 30% (V/V) hydrogen peroxide aqueous solution in 100 ml of TBS buffer] to form a color at a temperature of 25 °C for 10 minutes.

As such, similar procedures were performed on 4 groups, with 96 colonies per one group. In the two groups, protein band stained with luciferase anti-serum was recognized. Next, 96 colonies belonging to the two groups were divided into 8 groups with 12 colonies each and similar procedure was repeated. A protein that reacted with anti-luciferase serum was noted in one group. Finally, with respect to the 12 colonies contained in this group, each colony was treated in a similar manner, whereby a protein-producing colony that reacted with luciferase anti-serum was identified. By the foregoing procedure, 2 colonies containing luciferase c-DNA were obtained. From the two colonies, plasmid DNA was produced by the method described in Item 5. The obtained recombinant plasmid DNAs were named pALf2B8 and pALF3A6, respectively.

9. Survey of large luciferase c-DNA - Production of DNA probe

In 330 µl of TE buffer was dissolved 100 µg of recombinant plasmid pALF3A6 DNA. To the solution were added 40 µl of Low buffer [100 mM Tris-hydrochloride buffer (pH 7.5)/100 mM $MgCl_2$/10 mM dithiothreitol], 130 units of Pst I (manufactured by Takara Shuzo Co., Ltd.) and 120 units of Sac I (manufactured by Boehringer Mannheim GmbH) to effect cleavage at a temperature of 37 °C for 1.5 hours.

The whole amount of DNA was separated by electrophoresis using 0.7% (W/V) agarose gel. The agarose gel electrophoresis was carried out in accordance with the method of T. Maniatis et al., Molecular Cloning, pages 156-161, Cold Spring Harbor Laboratory (1984)]. DNA band containing luciferase c-DNA was excised and put in a dialysis tube. After 2 ml of TE buffer was supplemented, the dialysis tube was sealed and DNA was eluted from the gel into the buffer by electrophoresis. An equivalent volume of water-saturated phenol was added to this solution. After agitation, the aqueous phase was recovered and DNA was recovered by precipitation with ethanol in a conventional manner.

10 μg of the obtained DNA fragment was dissolved in 126 μl of TE buffer and 16 μl of Med buffer and 64 units of Sau 3 AI (manufactured by Takara Shuzo Co., Ltd.) were added to the solution. After reacting at a temperature of 37° C for 2 hours, the whole amount was subjected to electrophoresis using 5% (W/V) polyacrylamide gel thereby to isolate DNA fragments. The polyacrylamide gel electrophoresis was carried out in accordance with the method of A. Maxam [Methods in Enzymology, 65, 506 (1980)]. DNA fragment of 190 bp was isolated by the method as described above to give 1 μg of Sau3 AI luciferase c-DNA fragment.

Using [$\alpha$-$^{32}$P] dCTP (manufactured by Amersham Co.), 1 μg of this luciferase c-DNA was labeled according to the nick translation method. The nick translation method was performed using a kit manufactured by Takara Shuzo Co., Ltd. in accordance with the method described in J. Mol. Biol., 113, 237-251 (1977) and Molecular Cloning, pages 109-112, Cold Spring Harbor Laboratory (1982).

10. Survey of large luciferase c-DNA - Colony hybridization

Using as a probe the luciferase c-DNA fragment labelled with $^{32}$P produced by the method described above, c-DNA bank of the posterior portion of Photinus pyralis wherein recombinant plasmid pUC19 DNA was a vector was surveyed by colony hybridization [(Protein, Nucleic Acid and Enzyme, 26, 575-579 (1981)] to give colonies having luciferase c-DNA. Recombinant plasmid DNA possessed by one of the colonies was named pALF3 and plasmid DNA was produced by the method described in Item 5. E. coli containing the recombinant plasmid DNA was named E. coli DH 1 (pALf3). The transformant has been deposited as ATCC 67462.

The recombinant plasmid pALf3 DNA described above was subjected to single digestion and double digestion using Xba I, Hind III, BamH I, Eco RI and Pst I (all manufactured by Takara Shuzo Co., Ltd.). The obtained DNA fragments were analyzed by agarose gel electrophoresis on mobility pattern. By comparing the obtained mobility pattern with standard mobility pattern of DNA fragment obtained by digesting λ phage DNA (manufactured by Takara Shuzo Co., Ltd.) with Hind III, the size was turned out to be 1,700 bp. A restriction enzyme map of the plasmid described above is shown in Fig. 1.

11. Production of m-RNA of Luciola cruciata

Ten grams of living Luciola cruciata (GENJI firefly, purchased from Seibu Department Store) were put in a ultra-low temperature freezer box and frozen. Each posterior portion was cut off with scissors. To 2 g of the obtained posterior portion was added 18 ml of guanidine isothiocyanate solution. According to the method described in Item 1, 1.1 mg of RNA was produced. In accordance with the method described in Item 1, 1.1 mg of this RNA was subjected to column chromatography of oligo (dT)-cellulose to produce 30 μg of m-RNA for the posterior portion of Luciola cruciata.

12. Production of c-DNA bank of Luciola cruciata posterior portion

Synthesis of c-DNA was performed using a kit purchased from Amersham Co. in accordance with the method indicated by Amersham Co. which is described in Mol. Cell Biol., 2, 161 (1982) and Gene, 25, 263 (1983).

From 2 μg of the Luciola cruciata posterior portion RNA, 0.9 μg of double stranded c-DNA was synthesized. Using the method described in Item 4, a tail of polydeoxycytidine was added to 0.3 μg of this c-DNA.

This c-DNA, 20 ng, and 500 ng of pUC19 plasmid produced in Item 6, wherein a polyguanosine tail had been added to the Pst I site thereof, were annealed in accordance with the method described in Item 7. E. coli DH 1 strain (ATCC 33849) was transformed by annealed DNA by the method of Hanahan [DNA Cloning, 1, 109-135 (1985)] thereby to produce c-DNA bank of Luciola cruciata posterior portion.

13. Survey of luciferase c-DNA derived from Luciola cruciata

In 90 μl of TE buffer was dissolved 10 μg of recombinant plasmid pALF3 DNA obtained in Item 10 and, 10 μl of Med buffer, 25 units of restriction enzyme Eco RI and 25 units of restriction enzyme Cla I (both manufactured by Takara Shuzo Co., Ltd.) were added to the solution. The reaction was performed at a temperature of 37°C for 2 hours to cleave DNA. From the cleaved recombinant plasmid pALF3 DNA, 800 bp of Eco RI/Cla I DNA fragment containing luciferase c-DNA derived from Photinus pyralis (American firefly) was isolated in accordance with the method described in Item 9 using agarose gel electrophoresis. Thus, 1 μg of Eco RI/Cla I DNA fragment was obtained. Using [$\alpha$-$^{32}P$] dCTP (manufactured by Amersham Co.), 1 μg of this DNA was labelled with $^{32}P$ in accordance with the nick translation method described in Item 9. Using as a probe the Eco RI/Cla I DNA fragment labeled with $^{32}P$, c-DNA bank of the Luciola cruciata posterior portion was surveyed by the colony hybridization described in Item 10 thereby to select E. coli having luciferase c-DNA derived from Luciola cruciata. Several colonies of E. coli capable of hybridizing with the probe were obtained. Recombinant plasmid DNA possessed by one of these colonies was named pGLf1. The recombinant plasmid DNA was isolated in accordance with the method described in Item 5. E. coli containing the recombinant plasmid DNA was named E. coli DH1 (pGLf1). The transformant has been deposited as ATCC 67482.

The recombinant plasmid pGLf1 DNA described above was subjected to single digestion and double digestion using Hpa I, Hind III, Eco RV, Dra I, Af1 II, Hinc II, Pst I (all manufactured by Takara Shuzo Co., Ltd.) and Ssp I (manufactured by New England Biolab Co.). The obtained DNA fragments were analyzed by agarose gel electrophoresis on mobility pattern. By comparing the obtained mobility pattern with standard mobility pattern of DNA fragment obtained by digesting λ phage DNA (manufactured by Takara Shuzo Co., Ltd.) with Hind III, the size of the c-DNA inserted in pGLf1 was found to be 2,000 bp. A restriction enzyme map of the plasmid described above is shown in Fig. 2.

14. Analysis of base sequence of luciferase c-DNA derived from Luciola cruciata

Recombinant plasmid pGLf1 DNA, 10 μg, was cleaved with restriction enzyme Pst I (manufactured by Takara Shuzo Co., Ltd.) to give 2.5 μg of 2.0 Kb DNA fragment containing luciferase c-DNA. This DNA fragment was cloned at the Pst I site of plasmid pUC 119 DNA (manufactured by Takara Shuzo Co., Ltd.). The obtained plasmid DNAs were named pGLf2 and pGLf3, respectively, depending upon difference in the direction of inserting c-DNA. A cleavage treatment of recombinant plasmid pGLf1 DNA and plasmid pUC 119 DNA with Pst I (method described in Item 6), isolation of the luciferase c-DNA fragments using agarose gel electrophoresis (described in Item 9), ligation of plasmid pUC 119 DNA and luciferase c-DNA fragment (described in Item 5), transformation of Escherichia coli JM101 strain (ATCC 33876) using the ligation reaction solution (described in Item 5) and preparation of recombinant plasmid pGLf2 and pGLf3 DNAs described in Item 5) followed the methods described within parentheses.

Next, using the recombinant plasmid pGLf2 and pGLf3 DNAs, plasmid DNAs wherein various deletions were introduced into luciferase c-DNA were prepared using a deletion kit for killosequence (manufactured by Takara Shuzo Co., Ltd.) in accordance with the method of Henikoff [Gene, 28, 351-359 (1984)], followed by introducing into Escherichia coli JM101 strain (ATCC 33876) described in Item 5. By infecting the thus obtained Escherichia coli with helper phage M13K07 (manufactured by Takara Shuzo Co., Ltd.), single strand DNA was prepared in accordance with the method of Messing [Methods in Enzymology, 101, 20-78 (1983)]. Sequencing with the obtained single strand DNA was carried out by the method of Messing described above, using M13 sequencing kit (manufactured by Takara Shuzo Co., Ltd.). Gel electrophoresis for analyzing a base sequence was carried out using 8% (W/V) polyacrylamide gel (manufactured by Fuji Photo Film Co., Ltd.).

The base sequence of the luciferase-coding region of Luciola cruciata-derived luciferase c-DNA alone is shown in Figure 3. An amino acid sequence of the protein translated from the c-DNA is shown in Figure 4.

15. Construction of recombinant plasmid pGLf37 DNA

Firstly, production of DNA fragment containing the Luciola cruciata-derived luciferase gene deleted of the nucleotide sequence encoding 9 amino acids from the N-terminus and vector DNA is described. To a solution of 1 μg of recombinant plasmid pGLf1 DNA in 90 μl of water were added 10 μl of Med buffer and 20 units of Pst I (manufactured by Takara Shuzo Co., Ltd.). After digesting at a temperature of 37°C for 2

hours, an equivalent volume of water-saturated phenol was added thereto. Treatment for removing protein and precipitation with ethanol in a conventional manner were followed by ligation and transformation into Escherichia coli JM101 (ATCC 33876) according to the procedure described in Item 5.

DNA was isolated from the obtained transformants by the procedures described in Item 5. By digestion with restriction enzymes Ssp I, Eco RV and Pst I, etc. singly or doubly, a recombinant plasmid in which the direction of c-DNA was opposite the existing recombinant plasmid pGLf1, was selected and named pGLf10.

To a solution of 10 µg of recombinant plasmid pGLf10 DNA in 90 µl of water were added 10 µl of Med buffer and 10 units of Ssp I (manufactured by New England Biolab Co.). After digesting at a temperature of 37° C for 30 minutes, partially digested product was obtained. From the partially digested product, 2 µg of the 4.0 Kb DNA fragment containing the luciferase gene deleted of the nucleotide sequence encoding 9 amino acids from the N-terminus and most of the vector DNA was isolated.

Next, to a solution of 1 µg of the DNA fragment in 95 µl of water were added 5 µl of 1 M Tris-hydrochloride buffer (pH 8.0) and 0.3 units (1 µl) of alkaline phosphatase (manufactured by Takara Shuzo Co., Ltd.). After effecting at a temperature of 65° C for an hour, treatment for removing protein and precipitation with ethanol in a conventional manner gave precipitates. The both termini were dephosphorylated to give 1 µg of the DNA fragment of 4.0 Kb.

Next, preparation of the DNA fragment containing trp promoter derived from Escherichia coli.

To a solution of 10 µg of plasmid pKN206 DNA [Agric. Biol. Chem., 50, 271-279 (1986)] containing trp promoter in 90 µl of water were added 10 µl of Med buffer and 20 units of Cla I (manufactured by Takara Shuzo Co., Ltd.). After digesting at a temperature of 37° C for 2 hours, fully digested product was obtained. To the fully digested product were added 10 units of Ssp I described above. The mixture was treated at a temperature of 37° C for 30 minutes to give partially digested product with Ssp I. Treatment for removing protein and precipitation with ethanol in a conventional manner gave precipitates. The precipitates were dissolved in 100 µl of TE buffer and DNA fragment of 500 bp containing most of the trp promoter was isolated from the solution, according to the procedure described in Item 9.

Next, preparation of synthetic DNA is described below.

From the nucleotide sequence, it is assumed that the luciferase gene contained in the DNA fragment of 4.0 Kb described above would be deleted of the nucleotide sequence encoding 9 amino acids from the N-terminus.

Furthermore, the trp promoter contained in the DNA fragment of 500 bp described above is deleted of a part of the nucleotide sequence between SD and ATG. Therefore, in order to compensate for the nucleotide sequence encoding 9 amino acids from the N-terminus and the nucleotide sequence of trp promoter between SD and ATG, the following two synthetic DNAs were synthesized using System 1E Plus DNA Synthesizer manufactured by Beckmann Inc.

5′ CGACAATGGAAAACATGGAAAACGATGAAAAT 3′

5′ ATTTTCATCGTTTTCCATGTTTTCCATTGT 3′

By applying these two synthetic DNAs to NENSORB PREP manufactured by Du Pont Corp., 20 µg each of the purified synthetic DNAs were obtained. One microgram each of the synthetic DNAs was dissolved in 45 µl of water, respectively and 5 µl of 10-fold kination buffer [0.5 M Tris-hydrochloride buffer (pH 7.6)/0.1 M $MgCl_2$/50 mM dithiothreitol/10 mM ATP] was added to the solution. After further adding 10 units (1 µl) of T4 polynucleotide kinase (manufactured by Takara Shuzo Co., Ltd.) thereto, the mixture was treated at a temperature of 37° C for an hour followed by treatment for removing protein and precipitation with ethanol in a conventional manner. Thus, 1 µg each of synthetic DNAs phosphorylated at the 5′ end thereof was obtained, respectively.

Next, the desired plasmid DNA was obtained by ligation.

In 8 µl each of water were dissolved 1 µg of the aforesaid dephosphorylated DNA fragment of 4.0 Kb containing the luciferase gene deleted of the nucleotide sequence encoding 9 amino acids from the N-terminus and vector DNA, 1 µg of the aforesaid 500 bp DNA fragment containing trp promoter and 0.1 µg of the aforesaid two synthetic DNAs phosphorylated, respectively. After 1 µl of 10-fold ligation buffer [200 mM $MgCl_2$/660 mM Tris-hydrochloride buffer (pH 7.6)/10 mM ATP/150 mM dithiothreitol] and 1 unit (1 µl) of T4 DNA ligase (manufactured by Takara Shuzo Co., Ltd.) were added to each solution obtained, each mixture was treated at a temperature of 16° C for 16 hours. Using the reaction solution, transformation into Escherichia coli JM101 strain (ATCC 33876) was performed by the procedures described in Item 5. Plasmid DNA was isolated from the obtained transformants by the procedures described in Item 5. After digestion with restriction enzymes Ssp I, EcoR V and Pst I, etc. singly or doubly, the digestion product was developed by 0.7% agarose gel electrophoresis to obtain a plasmid capable of expressing the luciferase gene fully encoding luciferase gene by trp promoter. The recombinant plasmid was named pGLf37 and Escherichia coli containing the plasmid was named Escherichia coli JM101 strain (pGLf37).

16. Construction of recombinant plasmid pGLf15 DNA

To a solution of 5 µg of recombinant plasmid pGLf1 DNA in 90 µl of TE buffer were added 10 µl of Med buffer and 25 units of Ssp I . After digesting at a temperature of 37° C for 2 hours, an equivalent volume of water-saturated phenol was added thereto. Treatment for removing protein was performed in a conventional manner. From the digested recombinant plasmid pGLf1 DNA, the 1.7 Kb DNA fragment encoding luciferase c-DNA derived from Luciola cruciata was isolated by the method using agarose gel electrophoresis described in Item 9 to obtain 1 µg of the 1.7 Kb Ssp I fragment.

On the other hand, 1 µg of plasmid pUC 18 DNA (manufactured by Takara Shuzo Co., Ltd.) was dissolved in 18 µl of TE buffer and, 2 µl of Sma I buffer [100 mM Tris-hydrochloride buffer (pH 8.0)/70 mM magnesium chloride/200 mM potassium chloride/70 mM 2-mercaptoethanol/0.1% bovine serum albumin] and 5 units of Sma I (manufactured by Takara Shuzo Co., Ltd.) were added to the solution. After digesting at a temperature of 37° C for an hour, extraction with phenol and precipitation with ethanol were performed in a conventional manner to give precipitates.

In 7 µl of water were dissolved 0.5 µg of the Sma I-digested plasmid pUC 18 DNA 0.5 µg of 1.7 Kb Luciola cruciata-derived luciferase c-DNA fragment. To the solution was added 13 µl of a mixture [77 mM Tris-hydrochloride buffer (pH 7.4)/15 mM magnesium chloride/15 mM dithiothreitol/0.15 mM adenosine triphosphate] and 1 unit of T4 ligase (manufactured by Boehringer Mannheim AG). The mixture was subjected to ligation at a temperature of 8° C for 18 hours. Using the reaction solution, Escherichia coli JM101 strain (ATCC 33876) was transformed as described in Item 7. From the obtained transformants, plasmid DNA was isolated as described in Item 5. The isolated plasmid DNA was subjected to single digestion with Hind III (manufactured by Takara Shuzo Co., Ltd.) and plasmid DNA providing 1.5 Kb and 2.9 Kb DNA fragments was selected. This recombinant plasmid DNA was named pGLf15 and Escherichia coli containing said plasmid DNA was named Escherichia coli JM101 (pGLf15). Escherichia coli JM101 (pGLf15) has been deposited as ATCC 67461.

Escherichia coli JM101 (pGLf15) was cultured by the method described in Item 5. By isolating the recombinant plasmid DNA, 1.2 mg of purified recombinant pGLf15 DNA was obtained from 1 liter of the culture solution.

17. Cultivation of Escherichia coli JM101 (pGLf37) and preparation of crude enzyme solution

Escherichia coli JM101 (pGLf37) was shake cultured in 3 ml of LB-amp medium [1% (W/V) bactotrypton, 0.5% (W/V) yeast extract, 0.5% (W/V) NaCl and ampicillin (50 µg/ml)] at a temperature of 37° C for 18 hours. This culture solution, 0.5 ml, was inoculated on 10 ml of the aforesaid LB-amp medium. After shake cultured at a temperature of 37° C for 4 hours, the culture was subjected to a centrifuging operation at 8,000 r.p.m. for 10 minutes to give 20 mg of wet cells.

The recovered cells were suspended in 0.9 ml of buffer composed of 0.1 M $KH_2PO_4$ (pH 7.8), 2 mM EDTA, 1 mM dithiothreitol and 0.2 mg/ml protamine sulfate. Further 100 µl of 10 mg/ml lysozyme solution was supplemented to the suspension. The mixture was allowed to stand in ice for 15 minutes. Next, the suspension was frozen in methanol-dry ice bath and then allowed to stand at a temperature of 25° C to completely thaw. Further by performing a centrifuging operation at 12,000 r.p.m. for 5 minutes, 1 ml of crude enzyme solution was obtained as the supernatant (present invention).

The luciferase activity in the thus obtained crude enzyme solution was determined by the method described below. The results are shown in the table below.

The measurement of luciferase activity in the crude enzyme solution obtained was performed by counting the number of photon formed in accordance with the method of Kricka [Archives of Biochemistry and Biophysics, 217, 674 (1982)].

That is, 260 µl of 25 mM glycylglycine buffer (pH 7.8), 16 µl of 0.1 M magnesium sulfate and 24 µl of 1 mM luciferine (manufactured by Sigma Co.) and 10 µl of the crude enzyme solution were mixed. Then 100 µl of 20 mM ATP was added to the mixture. The number of photon formed was integrated for 20 seconds. The integrated values are shown in the table below.

For purpose of comparison, Escherichia coli JM101 (pGLf15) (ATCC 67461) was shake cultured in 3 ml of LB-amp medium [1% (W/V) bactotrypton, 0.5% (W/V) yeast extract, 0.5% (W/V) NaCl and ampicillin (50 µg/ml)] at a temperature of 37° C for 18 hours. This culture solution, 0.5 ml, was inoculated on 10 ml of the aforesaid LB-amp medium and 1 mM isopropyl-β-D-thiogalactoside was added thereto. After shake culture at a temperature of 37° C for 4 hours, the culture was subjected to a centrifuging operation at 8,000 r.p.m. for 10 minutes to give 20 mg of wet cells.

The recovered cells were suspended in 0.9 ml of buffer composed of 0.1 M $KH_2PO_4$ (pH 7.8), 2 mM EDTA, 1 mM dithiothreitol and 0.2 mg/ml protamine sulfate. Further 100 µl of 10 mg/ml lysozyme solution was supplemented to the suspension. The mixture was allowed to stand in ice for 15 minutes. Next, the suspension was frozen in methanol-dry ice bath and then allowed to stand at a temperature of 25°C to completely thaw. Further by performing a centrifuging operation at 12,000 r.p.m. for 5 minutes, 1 ml of crude enzyme solution was obtained as the supernatant (U.S. Patent Application Serial No. 224,445).

The luciferase activity in the thus obtained crude enzyme solution was determined by the method deescribed above. The results are shown in the table below.

For purpose of further comparison, luciferase activity was measured also with plasmid pUC18 DNA-bearing Escherichia coli JM101 strain [Escherichia coli JM101 (pUC 18)]. The results are shown in the table below as control.

Table

| Sample \ Item | Number of Photon/ml Culture Solution |
|---|---|
| Escherichia coli JM101 (pGLf37) (invention) | $3.0 \times 10^8$ |
| Escherichia coli JM101 (pGLf15) (U.S. Patent Application Serial No. 224,445) | $8.3 \times 10^6$ |
| Escherichia coli JM101 (pUC 18) (control) | $9.8 \times 10^4$ |

As is clear from the table above, it is noted that the count of photon increased in the present invention as compared to the prior inventions and to the control. It is thus noted that marked amounts of luciferase are produced in the cells of Escherichia coli used in the present invention.

## Claims

1. A method for producing luciferase which comprises culturing in a medium a luciferase-producing microorganism belonging to the genus Escherichia and containing a recombinant DNA having incorporated into a vector DNA a luciferase gene encoding an amino acid sequence shown below, and collecting luciferase from the culture.

| | | | | | | | | | |
|---|---|---|---|---|---|---|---|---|---|
| | | | | | | | | | 10 |
| Met | Glu | Asn | Met | Glu | Asn | Asp | Glu | Asn | Ile |
| | | | | | | | | | 20 |
| Val | Val | Gly | Pro | Lys | Pro | Phe | Tyr | Pro | Ile |
| | | | | | | | | | 30 |
| Glu | Glu | Gly | Ser | Ala | Gly | Thr | Gln | Leu | Arg |
| | | | | | | | | | 40 |
| Lys | Tyr | Met | Glu | Arg | Tyr | Ala | Lys | Leu | Gly |
| | | | | | | | | | 50 |
| Ala | Ile | Ala | Phe | Thr | Asn | Ala | Val | Thr | Gly |
| | | | | | | | | | 60 |
| Val | Asp | Tyr | Ser | Tyr | Ala | Glu | Tyr | Leu | Glu |
| | | | | | | | | | 70 |
| Lys | Ser | Cys | Cys | Leu | Gly | Lys | Ala | Leu | Gln |
| | | | | | | | | | 80 |
| Asn | Tyr | Gly | Leu | Val | Val | Asp | Gly | Arg | Ile |
| | | | | | | | | | 90 |
| Ala | Leu | Cys | Ser | Glu | Asn | Cys | Glu | Glu | Phe |

100
Phe Ile Pro Val Ile Ala Gly Leu Phe Ile

110
Gly Val Gly Val Ala Pro Thr Asn Glu Ile

120
Tyr Thr Leu Arg Glu Leu Val His Ser Leu

130
Gly Ile Ser Lys Pro Thr Ile Val Phe Ser

140
Ser Lys Lys Gly Leu Asp Lys Val Ile Thr

150
Val Gln Lys Thr Val Thr Thr Ile Lys Thr

160
Ile Val Ile Leu Asp Ser Lys Val Asp Tyr

170
Arg Gly Tyr Gln Cys Leu Asp Thr Phe Ile

180
Lys Arg Asn Thr Pro Pro Gly Phe Gln Ala

190
Ser Ser Phe Lys Thr Val Glu Val Asp Arg

200
Lys Glu Gln Val Ala Leu Ile Met Asn Ser

210
Ser Gly Ser Thr Gly Leu Pro Lys Gly Val

220
Gln Leu Thr His Glu Asn Thr Val Thr Arg

230
Phe Ser His Ala Arg Asp Pro Ile Tyr Gly

240
Asn Gln Val Ser Pro Gly Thr Ala Val Leu

250
Thr Val Val Pro Phe His His Gly Phe Gly

260
Met Phe Thr Thr Leu Gly Tyr Leu Ile Cys

270
Gly Phe Arg Val Val Met Leu Thr Lys Phe

280
Asp Glu Glu Thr Phe Leu Lys Thr Leu Gln

290
Asp Tyr Lys Cys Thr Ser Val Ile Leu Val

| | | | | | | | | | |
|---|---|---|---|---|---|---|---|---|---|
| | | | | | | | | | 300 |
| Pro | Thr | Leu | Phe | Ala | Ile | Leu | Asn | Lys | Ser |
| | | | | | | | | | 310 |
| Glu | Leu | Leu | Asn | Lys | Tyr | Asp | Leu | Ser | Asn |
| | | | | | | | | | 320 |
| Leu | Val | Glu | Ile | Ala | Ser | Gly | Gly | Ala | Pro |
| | | | | | | | | | 330 |
| Leu | Ser | Lys | Glu | Val | Gly | Glu | Ala | Val | Ala |
| | | | | | | | | | 340 |
| Arg | Arg | Phe | Asn | Leu | Pro | Gly | Val | Arg | Gln |
| | | | | | | | | | 350 |
| Gly | Tyr | Gly | Leu | Thr | Glu | Thr | Thr | Ser | Ala |
| | | | | | | | | | 360 |
| Ile | Ile | Ile | Thr | Pro | Glu | Gly | Asp | Asp | Lys |
| | | | | | | | | | 370 |
| Pro | Gly | Ala | Ser | Gly | Lys | Val | Val | Pro | Leu |
| | | | | | | | | | 380 |
| Phe | Lys | Ala | Lys | Val | Ile | Asp | Leu | Asp | Thr |
| | | | | | | | | | 390 |
| Lys | Lys | Ser | Leu | Gly | Pro | Asn | Arg | Arg | Gly |
| | | | | | | | | | 400 |
| Glu | Val | Cys | Val | Lys | Gly | Pro | Met | Leu | Met |
| | | | | | | | | | 410 |
| Lys | Gly | Tyr | Val | Asn | Asn | Pro | Glu | Ala | Thr |
| | | | | | | | | | 420 |
| Lys | Glu | Leu | Ile | Asp | Glu | Glu | Gly | Trp | Leu |
| | | | | | | | | | 430 |
| His | Thr | Gly | Asp | Ile | Gly | Tyr | Tyr | Asp | Glu |
| | | | | | | | | | 440 |
| Glu | Lys | His | Phe | Phe | Ile | Val | Asp | Arg | Leu |
| | | | | | | | | | 450 |
| Lys | Ser | Leu | Ile | Lys | Tyr | Lys | Gly | Tyr | Gln |
| | | | | | | | | | 460 |
| Val | Pro | Pro | Ala | Glu | Leu | Glu | Ser | Val | Leu |
| | | | | | | | | | 470 |
| Leu | Gln | His | Pro | Ser | Ile | Phe | Asp | Ala | Gly |
| | | | | | | | | | 480 |
| Val | Ala | Gly | Val | Pro | Asp | Pro | Val | Ala | Gly |
| | | | | | | | | | 490 |
| Glu | Leu | Pro | Gly | Ala | Val | Val | Val | Leu | Glu |

| | | | | | | | | | |
|---|---|---|---|---|---|---|---|---|---|
| | | | | | | | | | 500 |
| Ser | Gly | Lys | Asn | Met | Thr | Glu | Lys | Glu | Val |
| | | | | | | | | | 510 |
| Met | Asp | Tyr | Val | Ala | Ser | Gln | Val | Ser | Asn |
| | | | | | | | | | 520 |
| Ala | Lys | Arg | Leu | Arg | Gly | Gly | Val | Arg | Phe |
| | | | | | | | | | 530 |
| Val | Asp | Glu | Val | Pro | Lys | Gly | Leu | Thr | Gly |
| | | | | | | | | | 540 |
| Lys | Ile | Asp | Gly | Arg | Ala | Ile | Arg | Glu | Ile |
| Leu | Lys | Lys | Pro | Val | Ala | Lys | Met | | |

2. A method for producing luciferase as claimed in claim 1, wherein said luciferase gene is represented by a nucleotide sequence shown below.

| | | | |
|---|---|---|---|
| 10 | 20 | 30 | 40 |
| ATGGAAAACA | TGGAAAACGA | TGAAAATATT | GTAGTTGGAC |
| 50 | 60 | 70 | 80 |
| CTAAACCGTT | TTACCCTATC | GAAGAGGGAT | CTGCTGGAAC |
| 90 | 100 | 110 | 120 |
| ACAATTACGC | AAATACATGG | AGCGATATGC | AAAACTTGGC |
| 130 | 140 | 150 | 160 |
| GCAATTGCTT | TTACAAATGC | AGTTACTGGT | GTTGATTATT |
| 170 | 180 | 190 | 200 |
| CTTACGCCGA | ATACTTGGAG | AAATCATGTT | GTCTAGGAAA |
| 210 | 220 | 230 | 240 |
| AGCTTTGCAA | AATTATGGTT | TGGTTGTTGA | TGGCAGAATT |
| 250 | 260 | 270 | 280 |
| GCGTTATGCA | GTGAAAACTG | TGAAGAATTT | TTTATTCCTG |
| 290 | 300 | 310 | 320 |
| TAATAGCCGG | ACTGTTTATA | GGTGTAGGTG | TTGCACCCAC |
| 330 | 340 | 350 | 360 |
| TAATGAGATT | TACACTTTAC | GTGAACTGGT | TCACAGTTTA |
| 370 | 380 | 390 | 400 |
| GGTATCTCTA | AACCAACAAT | TGTATTTAGT | TCTAAAAAAG |
| 410 | 420 | 430 | 440 |
| GCTTAGATAA | AGTTATAACA | GTACAGAAAA | CAGTAACTAC |

450 460 470 480
TATTAAAACC ATTGTTATAC TAGATAGCAA AGTTGATTAT

490 500 510 520
CGAGGATATC AATGTCTGGA CACCTTTATA AAAAGAAACA

530 540 550 560
CTCCACCAGG TTTTCAAGCA TCCAGTTTCA AAACTGTGGA

570 580 590 600
AGTTGACCGT AAAGAACAAG TTGCTCTTAT AATGAACTCT

610 620 630 640
TCGGGTTCTA CCGGTTTGCC AAAAGGCGTA CAACTTACTC

650 660 670 680
ACGAAAATAC AGTCACTAGA TTTTCTCATG CTAGAGATCC

690 700 710 720
GATTTATGGT AACCAAGTTT CACCAGGCAC CGCTGTTTTA

730 740 750 760
ACTGTCGTTC CATTCCATCA TGGTTTTGGT ATGTTCACTA

770 780 790 800
CTCTAGGGTA TTTAATTTGT GGTTTTCGTG TTGTAATGTT

810 820 830 840
AACAAAATTC GATGAAGAAA CATTTTTAAA AACTCTACAA

850 860 870 880
GATTATAAAT GTACAAGTGT TATTCTTGTA CCGACCTTGT

890 900 910 920
TTGCAATTCT CAACAAAAGT GAATTACTCA ATAAATACGA

930 940 950 960
TTTGTCAAAT TTAGTTGAGA TTGCATCTGG CGGAGCACCT

970 980 990 1000
TTATCAAAAG AAGTTGGTGA AGCTGTTGCT AGACGCTTTA

1010 1020 1030 1040
ATCTTCCCGG TGTTCGTCAA GGTTATGGTT TAACAGAAAC

1050 1060 1070 1080
AACATCTGCC ATTATTATTA CACCAGAAGG AGACGATAAA

1090 1100 1110 1120
CCAGGAGCTT CTGGAAAAGT CGTGCCGTTG TTTAAAGCAA

1130 1140 1150 1160
AAGTTATTGA TCTTGATACC AAAAAATCTT TAGGTCCTAA

1170 1180 1190 1200
CAGACGTGGA GAAGTTTGTG TTAAAGGACC TATGCTTATG

1210 1220 1230 1240
AAAGGTTATG TAAATAATCC AGAAGCAACA AAAGAACTTA

```
         1250       1260       1270       1280
TTGACGAAGA AGGTTGGCTG CACACCGGAG ATATTGGATA
         1290       1300       1310       1320
TTATGATGAA GAAAAACATT TCTTTATTGT CGATCGTTTG
         1330       1340       1350       1360
AAGTCTTTAA TCAAATACAA AGGATACCAA GTACCACCTG
         1370       1380       1390       1400
CCGAATTAGA ATCCGTTCTT TTGCAACATC CATCTATCTT
         1410       1420       1430       1440
TGATGCTGGT GTTGCCGGCG TTCCTGATCC TGTAGCTGGC
         1450       1460       1470       1480
GAGCTTCCAG GAGCCGTTGT TGTACTGGAA AGCGGAAAAA
         1490       1500       1510       1520
ATATGACCGA AAAAGAAGTA ATGGATTATG TTGCAAGTCA
         1530       1540       1550       1560
AGTTTCAAAT GCAAAACGTT TACGTGGTGG TGTTCGTTTT
         1570       1580       1590       1600
GTGGATGAAG TACCTAAAGG TCTTACTGGA AAAATTGACG
         1610       1620       1630       1640
GCAGAGCAAT TAGAGAAATC CTTAAGAAAC CAGTTGCTAA

GATG
```

FIG. 1

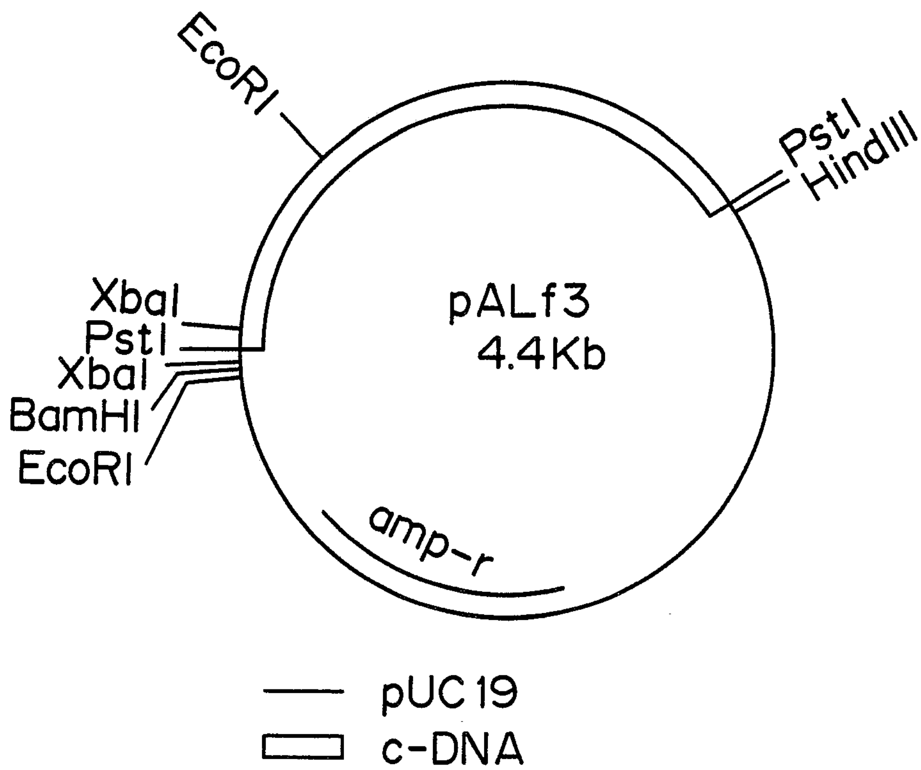
EcoRI
PstI
HindIII
XbaI
PstI
XbaI
BamHI
EcoRI
pALf3
4.4Kb
amp-r
pUC19
c-DNA

FIG. 2

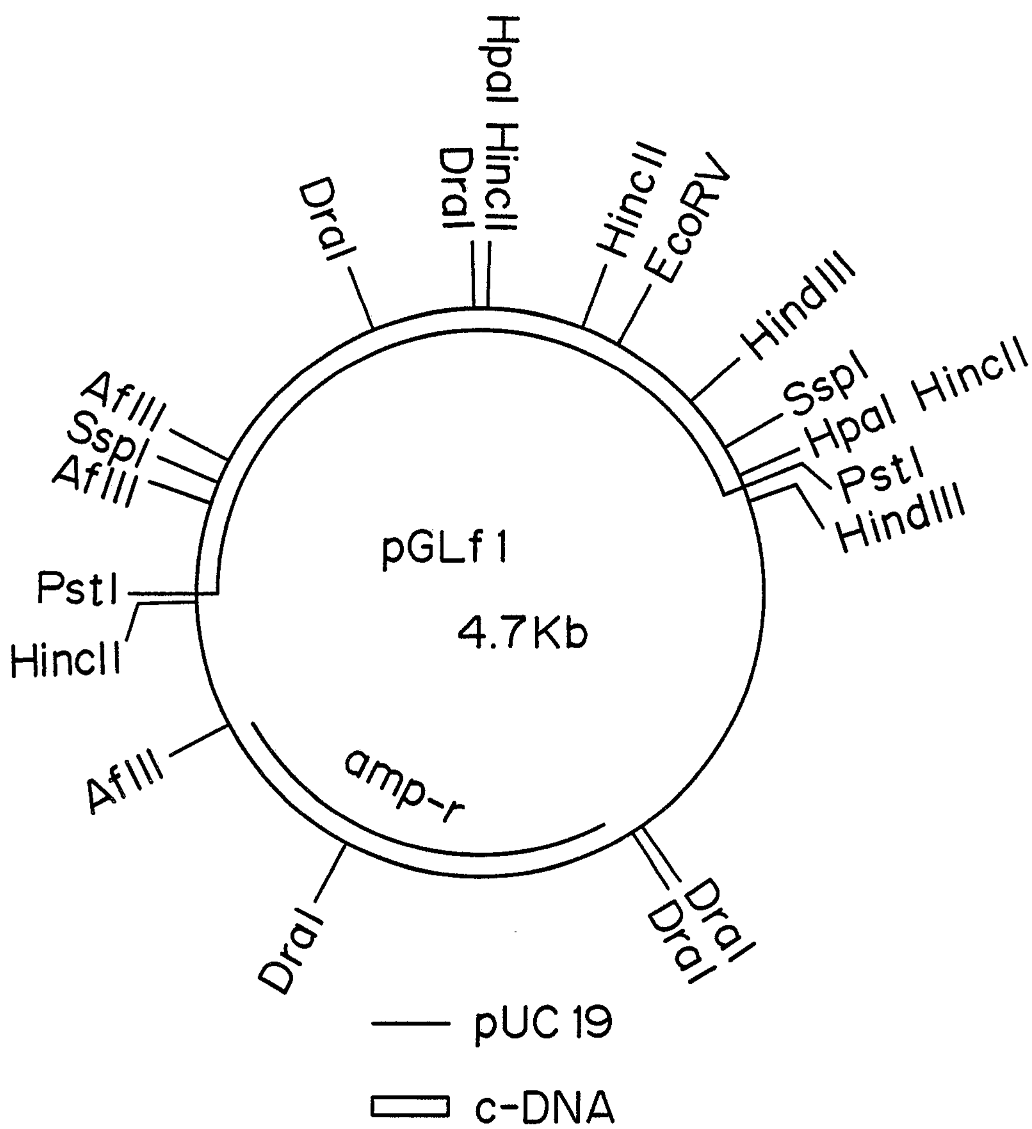
Hpal HincII
DraI
DraI
HincII
EcoRV
HindIII
SspI
Hpal HincII
PstI
HindIII
AfIII
SspI
AfIII
PstI
HincII
pGLf1
4.7Kb
amp-r
AfIII
DraI
DraI
DraI
pUC 19
c-DNA

FIG. 3A

| 10 | 20 | 30 | 40 | 50 | 60 |
|---|---|---|---|---|---|
| ATGGAAAACA | TGGAAAACGA | TGAAAATATT | GTAGTTGGAC | CTAAACCGTT | TTACCCTATC |
| 70 | 80 | 90 | 100 | 110 | 120 |
| GAAGAGGGAT | CTGCTGGAAC | ACAATTACGC | AAATACATGG | AGCGATATGC | AAAACTTGGC |
| 130 | 140 | 150 | 160 | 170 | 180 |
| GCAATTGCTT | TTACAAATGC | AGTTACTGGT | GTTGATTATT | CTTACGCCGA | ATACTTGGAG |
| 190 | 200 | 210 | 220 | 230 | 240 |
| AAATCATGTT | GTCTAGGAAA | AGCTTTGCAA | AATTATGGTT | TGGTTGTTGA | TGGCAGAATT |
| 250 | 260 | 270 | 280 | 290 | 300 |
| GCGTTATGCA | GTGAAAACTG | TGAAGAATTT | TTTATTCCTG | TAATAGCCGG | ACTGTTTATA |
| 310 | 320 | 330 | 340 | 350 | 360 |
| GGTGTAGGTG | TTGCACCCAC | TAATGAGATT | TACACTTTAC | GTGAACTGGT | TCACAGTTTA |
| 370 | 380 | 390 | 400 | 410 | 420 |
| GGTATCTCTA | AACCAACAAT | TGTATTTAGT | TCTAAAAAAG | GCTTAGATAA | AGTTATAACA |
| 430 | 440 | 450 | 460 | 470 | 480 |
| GTACAGAAAA | CAGTAACTAC | TATTAAAACC | ATTGTTATAC | TAGATAGCAA | AGTTGATTAT |
| 490 | 500 | 510 | 520 | 530 | 540 |
| CGAGGATATC | AATGTCTGGA | CACCTTTATA | AAAAGAAACA | CTCCACCAGG | TTTTCAAGCA |
| 550 | 560 | 570 | 580 | 590 | 600 |
| TCCAGTTTCA | AAACTGTGGA | AGTTGACCGT | AAAGAACAAG | TTGCTCTTAT | AATGAACTCT |
| 610 | 620 | 630 | 640 | 650 | 660 |
| TCGGGTTCTA | CCGGTTTGCC | AAAAGGCGTA | CAACTTACTC | ACGAAAATAC | AGTCACTAGA |
| 670 | 680 | 690 | 700 | 710 | 720 |
| TTTTCTCATG | CTAGAGATCC | GATTTATGGT | AACCAAGTTT | CACCAGGCAC | CGCTGTTTTA |
| 730 | 740 | 750 | 760 | 770 | 780 |
| ACTGTCGTTC | CATTCCATCA | TGGTTTTGGT | ATGTTCACTA | CTCTAGGGTA | TTTAATTTGT |
| 790 | 800 | 810 | 820 | 830 | 840 |
| GGTTTTCGTG | TTGTAATGTT | AACAAAATTC | GATGAAGAAA | CATTTTTAAA | AACTCTACAA |

FIG. 3B

```
       850        860        870        880        890        900
GATTATAAAT GTACAAGTGT TATTCTTGTA CCGACCTTGT TTGCAATTCT CAACAAAAGT
       910        920        930        940        950        960
GAATTACTCA ATAAATACGA TTTGTCAAAT TTAGTTGAGA TTGCATCTGG CGGAGCACCT
       970        980        990       1000       1010       1020
TTATCAAAAG AAGTTGGTGA AGCTGTTGCT AGACGCTTTA ATCTTCCCGG TGTTCGTCAA
      1030       1040       1050       1060       1070       1080
GGTTATGGTT TAACAGAAAC AACATCTGCC ATTATTATTA CACCAGAAGG AGACGATAAA
      1090       1100       1110       1120       1130       1140
CCAGGAGCTT CTGGAAAAGT CGTGCCGTTG TTTAAAGCAA AAGTTATTGA TCTTGATACC
      1150       1160       1170       1180       1190       1200
AAAAAATCTT TAGGTCCTAA CAGACGTGGA GAAGTTTGTG TTAAAGGACC TATGCTTATG
      1210       1220       1230       1240       1250       1260
AAAGGTTATG TAAATAATCC AGAAGCAACA AAAGAACTTA TTGACGAAGA AGGTTGGCTG
      1270       1280       1290       1300       1310       1320
CACACCGGAG ATATTGGATA TTATGATGAA GAAAAACATT TCTTTATTGT CGATCGTTTG
      1330       1340       1350       1360       1370       1380
AAGTCTTTAA TCAAATACAA AGGATACCAA GTACCACCTG CCGAATTAGA ATCCGTTCTT
      1390       1400       1410       1420       1430       1440
TTGCAACATC CATCTATCTT TGATGCTGGT GTTGCCGGCG TTCCTGATCC TGTAGCTGGC
      1450       1460       1470       1480       1490       1500
GAGCTTCCAG GAGCCGTTGT TGTACTGGAA AGCGGAAAAA ATATGACCGA AAAAGAAGTA
      1510       1520       1530       1540       1550       1560
ATGGATTATG TTGCAAGTCA AGTTTCAAAT GCAAAACGTT TACGTGGTGG TGTTCGTTTT
      1570       1580       1590       1600       1610       1620
GTGGATGAAG TACCTAAAGG TCTTACTGGA AAAATTGACG GCAGAGCAAT TAGAGAAATC
      1630       1640
CTTAAGAAAC CAGTTGCTAA GATG
```

# FIG. 4A

```
                                      10                                      20
Met Glu Asn Met Glu Asn Asp Glu Asn Ile Val Val Gly Pro Lys Pro Phe Tyr Pro Ile
                                      30                                      40
Glu Glu Gly Ser Ala Gly Thr Gln Leu Arg Lys Tyr Met Glu Arg Tyr Ala Lys Leu Gly
                                      50                                      60
Ala Ile Ala Phe Thr Asn Ala Val Thr Gly Val Asp Tyr Ser Tyr Ala Glu Tyr Leu Glu
                                      70                                      80
Lys Ser Cys Cys Leu Gly Lys Ala Leu Gln Asn Tyr Gly Leu Val Val Asp Gly Arg Ile
                                      90                                     100
Ala Leu Cys Ser Glu Asn Cys Glu Glu Phe Phe Ile Pro Val Ile Ala Gly Leu Phe Ile
                                     110                                     120
Gly Val Gly Val Ala Pro Thr Asn Glu Ile Tyr Thr Leu Arg Glu Leu Val His Ser Leu
                                     130                                     140
Gly Lle Ser Lys Pro Thr Ile Val Phe Ser Ser Lys Lys Gly Leu Asp Lys Val Ile Thr
                                     150                                     160
Val Gln Lys Thr Val Thr Thr Ile Lys Thr Ile Val Ile Leu Asp Ser Lys Val Asp Tyr
                                     170                                     180
Arg Gly Tyr Gln Cys Leu Asp Thr phe Ile Lys Arg Asn Thr Pro Pro Gly Phe Gln Ala
                                     190                                     200
Ser Ser Phe Lys Thr Val Glu Val Asp Arg Lys Glu Gln Val Ala Leu Ile Met Asn Ser
                                     210                                     220
Ser Gly Ser Thr Gly Leu Pro Lys Gly Val Gln Leu Thr His Glu Asn Thr Val Thr Arg
                                     230                                     240
Phe Ser His Ala Arg Asp Pro Ile Tyr Gly Asn Gln Val Ser Pro Gly Thr Ala Val Leu
                                     250                                     260
Thr Val Val Pro Phe His His Gly Phe Gly Met Phe Thr Thr Leu Gly Tyr Leu Ile Cys
                                     270                                     280
Gly Phe Arg Val Val Met Leu Thr Lys Phe Asp Glu Glu Thr Phe Leu Lys Thr Leu Gln
```

# FIG. 4B

```
                                       290                                    300
Asp Tyr Lys Cys Thr Ser Val Ile Leu Val Pro Thr Leu Phe Ala Ile Leu Asn Lys Ser
                                       310                                    320
Glu Leu Leu Asn Lys Tyr Asp Leu Ser Asn Leu Val Glu Ile Ala Ser Gly Gly Ala Pro
                                       330                                    340
Leu Ser Lys Glu Val Gly Glu Ala Val Ala Arg Arg Phe Asn Leu Pro Gly Val Arg Gln
                                       350                                    360
Gly Tyr Gly Leu Thr Glu Thr Thr Ser Ala Ile Ile Ile Thr Pro Glu Gly Asp Asp Lys
                                       370                                    380
Pro Gly Ala Ser Gly Lys Val Val Pro Leu Phe Lys Ala Lys Val Ile Asp Leu Asp Thr
                                       390                                    400
Lys Lys Ser Leu Gly Pro Asn Arg Arg Gly Glu Val Cys Val Lys Gly Pro Met Leu Met
                                       410                                    420
Lys Gly Tyr Val Asn Asn Pro Glu Ala Thr Lys Glu Leu Ile Asp Glu Glu Gly Trp Leu
                                       430                                    440
His Thr Gly Asp Ile Gly Tyr Tyr Asp Glu Glu Lys His Phe Phe Ile Val Asp Arg Leu
                                       450                                    460
Lys Ser Leu Ile Lys Tyr Lys Gly Tyr Gln Val Pro Pro Ala Glu Leu Glu Ser Val Leu
                                       470                                    480
Leu Gln His Pro Ser Ile Phe Asp Ala Gly Val Ala Gly Val Pro Asp Pro Val Ala Gly
                                       490                                    500
Glu Leu Pro Gly Ala Val Val Val Leu Glu Ser Gly Lys Asn Met Thr Glu Lys Glu Val
                                       510                                    520
Met Asp Tyr Val Ala Ser Gln Val Ser Asn Ala Lys Arg Leu Arg Gly Gly Val Arg Phe
                                       530                                    540
Val Asp Glu Val Pro Lys Gly Leu Thr Gly Lys Ile Asp Gly Arg Ala Ile Arg Glu Ile

Leu Lys Lys Pro Val Ala Lys Met
```

FIG. 5

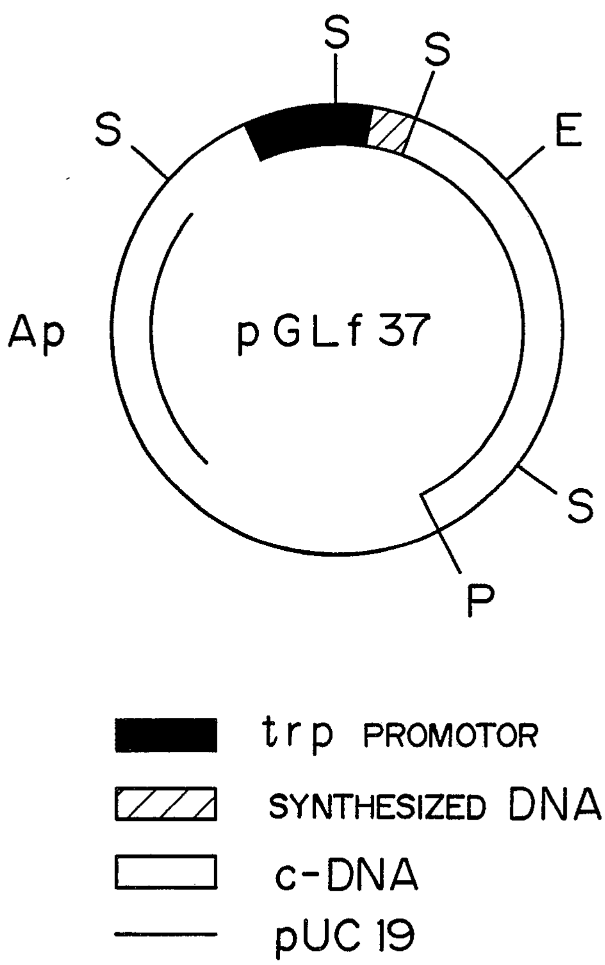

European Patent Office

# EUROPEAN SEARCH REPORT

Application Number

EP 89 11 6099

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | PROC. NATL. ACAD. SCI. USA, vol. 82, December 1985, pages 8780-7873; J.R. DE WET et al.: "Cloning of firefly luciferase cDNA and the expression of active luciferase in Escherichia coli" * The whole article * | 1-2 | C 12 N 15/53 |
| Y | GENE, vol. 54, no. 2-3, 1987, pages 203-210, Amsterdam, NL; E.F. DELONG et al.: "Isolation of the lux genes from Photobacterium leiognathi and expression in Escherichia coli" * The whole article * | 1-2 | |
| Y | WO-A-8 703 304 (THE REGENTS OF THE UNIVERSITY OF CALIFORNIA) * The whole document * | 1-2 | |
| P,X | GENE, vol. 77, 1989, pages 265-270; T. MASUDA et al.: "Cloning and sequence analysis of cDNA for luciferase of a Japanese firefly, Luciola cruciata" * The whole document * | 1-2 | TECHNICAL FIELDS SEARCHED (Int. Cl.5) |
| P,X | CHEMICAL ABSTRACTS, vol. 110, no. 13, 27th March 1989, page 174, abstract no. 109122g, Columbus, Ohio, US; E. NAKANO et al.: "Cloning of luciferase gene in Luciola cruciata", & BAIOSAIENSU TO INDASUTORI 1988, 46(5), 3190-2 * The whole abstract * | 1-2 | C 12 N |
| P,X | EP-A-0 301 541 (KIKKOMAN CORP.) * The whole document * | 1-2 | |

The present search report has been drawn up for all claims

| Place of search | Date of completion of the search | Examiner |
|---|---|---|
| THE HAGUE | 10-01-1990 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document